# EUROPEAN PATENT APPLICATION

(11) **EP 4 825 902 A1**
(43) Date of publication of application: **21.10.2026**
(21) Application number: 25170624.8
(22) Date of filing: 15.04.2025
(51) Int. Cl.: C08J 11/20, C07C 4/22, C07C 37/00

(54) **METHANOLYSIS OF AN ARTICLE COMPRISING POLYCARBONATE AND WHEREIN THE AT LEAST ONE VINYL(CO)POLYMER AND/OR RUBBER-MODIFIED GRAFT POLYMER IS IN THE LIQUID PHASE**

(71) Applicant: Covestro Deutschland AG, 51373 Leverkusen (DE)
(72) Inventor: YOGENDRA, Sivathmeehan, 40211 Düsseldorf (DE); SLUYTS, Erik, 2930 Brasschaat (BE); DE VYLDER, Anton, 9160B Eksaarde (BE); THIEL, Daniel, 51375 Leverkusen (DE); VANDEN EYNDE, Johan, 9052 Zwijnaarde (BE)
(74) Representative: Levpat

(57) **Abstract**

The present invention relates to a process for the recovery of a dihydroxy compound and optionally a vinyl(co)polymer and/or rubber-modified graft polymer from an article comprising polycarbonate and at least one vinyl(co)polymer and/or rubber-modified graft polymer by methanolysis, wherein the at least one vinyl(co)polymer and/or rubber-modified graft polymer is in the liquid state at least after preforming the methanolysis reaction. Moreover, the present invention relates to the production of a polymer by polymerizing the dihydroxy compound recovered by the inventive process and the production of a polymer blend by blending the vinyl(co)polymer and/or rubber-modified graft polymer recovered by the inventive process with a further polymer.

## Description

The present invention relates to a process for the recovery of a dihydroxy compound and optionally a vinyl(co)polymer and/or rubber-modified graft polymer from an article comprising polycarbonate and at least one vinyl(co)polymer and/or rubber-modified graft polymer by methanolysis, wherein the at least one vinyl(co)polymer and/or rubber-modified graft polymer is in the liquid phase at least after the methanolysis. Moreover, the present invention relates to the production of a polymer by polymerizing the dihydroxy compound recovered by the inventive process and the production of a polymer blend by blending the vinyl(co)polymer and/or rubber-modified graft polymer recovered by the inventive process with a further polymer.

Polycarbonates (PC) are applied diversly in industry and everyday life. They are known for their good property profile in terms of mechanical and optical properties, temperature resistance, and weathering stability. Due to this property profile, polycarbonates have long been used in a wide variety of indoor and outdoor applications, for example for the automotive sector, for rail vehicles, for the construction sector, for the electrical/electronics/IT sector, for battery housings for household appliances, for the lighting sector or for the medical technology sector. The property profile of polycarbonate may further be adjusted to the demands of the respective use via the selection of additional polymeric components added to the polycarbonate and the ranges of amounts in which these components are used in the compositions.

To increase notch resistance, in particular at low temperatures, blending partners having elastomeric properties are added to the polycarbonate as impact modifiers. The impact modifiers in turn may differ in the chemical composition of the elastic component or else the morphology thereof. An important class of impact modifiers are rubber-modified graft polymers. They can be blended with polycarbonate in a compounding process and a two-phase morphology is created. In most cases the phase morphology of these polycarbonate blends is such that polycarbonate forms the matrix phase and the rubber-modified graft polymer forms domains within the matrix phase.

Typically, such rubber-modified graft polymers can additionally comprise at least one vinyl(co)polymer which is not bound to the rubber. Amongst those, styrene-acrylonitrile copolymers (SAN) combined with polybutadiene-based rubber particles, commonly known as acrylonitrile-butadiene-styrene (ABS), is the most prominent component. It is used on a large scale as blending partner with incorporated impact modifier for polycarbonate compositions. In this combination the rubber particles improve notched impact resistance and further mechanical properties, while SAN facilitates the melt flowability of the resulting blend with polycarbonate and hence facilitates processing to give molded articles.

In order to increase the surface hardness, scratch resistance, chemical resistance (e.g. suncream resistance) and/or to reduce the birefringence of polycarbonate, acrylate polymers may be blended with the polycarbonate composition. Depending on the desired properties, polyalkyl (alkyl)acrylates and/or copolymers of alkyl (alkyl)acrylates can be applied. The most prominent acrylate polymer in this context is poly(methyl methacrylate) (PMMA).

Poly(methyl methacrylate) is known to have better optical properties than aromatic polycarbonates. For example, the optical transmission is higher due to its purely aliphatic character. For the same reason, weathering stability is also better. Components made of PMMA are harder and therefore less sensitive to scratches than bisphenol A-based polycarbonates (BPA-PC). However, the lower heat resistance and lower toughness of PMMA compared to BPA-PC are disadvantageous. In order to make use of a combination of the respective material properties of BPA-PC and PMMA, both materials are blended. In case transparent materials with the tuned property profile resulting from the combination of polycarbonate and poly(methyl methacrylate) are desired, copolymers of alkyl (alkyl)acrylates or a combination of poly(methyl methacrylate) and copolymers of alkyl (alkyl)acrylates are often used.

As a result of the range of application possibilities due to a versatile and adjustable property profile, and the associated economic success, large quantities of waste comprising at least polycarbonate are generated. Such polycarbonate waste which may comprise additional blend partners must be put to meaningful use. The technically simplest type of use is incineration, utilizing the released combustion heat for other processes, such as industrial manufacturing processes. However, this method does not allow for the closure of raw material cycles. Another type of use is typically subsumed under the expression "physical recycling," in which polycarbonate waste is mechanically shredded and used in the production of new products. This type of recycling naturally has its limits, which is why there have been numerous attempts to recover the raw materials underlying polycarbonate production by cleaving the polycarbonate bonds (typically referred to as "chemical recycling"). The raw materials to be recovered usually include bisphenols, especially bisphenol A. Depending on the type of chemical recycling, a carbonate-containing compound such as diphenyl carbonate or dimethyl carbonate, or even CO₂, can also be obtained. In case of compositions comprising polycarbonate and at least one polymeric blend partner such as for example ABS or PMMA this polymeric blend partner would be also a valuable raw material and thus, it would be desirable to recycle it.

WO 2014/099548 A1 describes a method for alcoholysis of a composition comprising polycarbonate and a flame retardant, acrylonitrile-butadiene-styrene, or a combination of the flame retardant and acrylonitrile-butadiene-styrene. The method comprises contacting the composition with a solvent that forms a solution or a filterable suspension of the flame retardant and / or acrylonitrile-butadiene-styrene, but the polycarbonate is not dissolved; separating the solution or the filterable suspension from the polycarbonate and depolymerizing the separated polycarbonate in a next step. This means that the acrylonitrile-butadiene-styrene is not present during the methanolysis.

WO 2014/099594 A1 relates to a method for recovering a dihydroxy aromatic compound and a dialkyl carbonate from a composition comprising a polycarbonate and acrylonitrile-butadiene-styrene. The method comprises depolymerizing the polycarbonate producing a dihydroxy aromatic compound and a dialkyl carbonate; removing the dihydroxy aromatic compound and the dialkyl carbonate from the reactor, the acrylonitrile-butadiene-styrene remaining as a coating on the surfaces of the reactor; adding a solvent to the reactor; and heating the solvent to remove the acrylonitrile-butadiene-styrene from the surfaces of the reactor. The acrylonitrile-butadiene-styrene is present in solid form during the methanolysis.

In JP 4575046 B2 a method is described which is used for obtaining an aromatic dihydroxy compound metal salt aqueous solution from a laminate having a film of a resin other than an aromatic polycarbonate, e.g. melamine, acrylic, methacrylic or organopolysiloxane resins, laminated on an aromatic polycarbonate molded product. The method comprises the steps of dissolving the laminate in chlorinated organic solvent and separating the undissolved material comprising the film material before decomposing the aromatic polycarbonate dissolved in the chlorinated organic solvent in the presence of sodium hydroxide aqueous solution into an aromatic dihydroxy compound.

WO 2023/110654 A1 describes a process for the recovery of a rubber-modified vinyl(co)polymer from a polycarbonate resin comprising polycarbonate and a rubber-modified vinyl(co)polymer. The method comprises the step of bringing the polycarbonate resin into contact with an alcohol and a transesterification catalyst to obtain a slurry. By keeping the temperature below the softening temperature of the rubber-modified vinyl(co)polymer, i.e. at 60 to 90 °C, the rubber-modified vinyl(co)polymer is present as hard particles, which may be separated by filtration from the liquid fraction of the slurry comprising the diol resulting from the depolymerization of the polycarbonate.

WO 2023/163026 A1 relates a method for producing a bisphenol by decomposition of a polycarbonate resin in a reaction solution containing the polycarbonate resin, dialkyl carbonate, an aliphatic monoalcohol and a catalyst. The molar ratio of the dialkyl carbonate relative to 1 mol of repeating unit derived from the bisphenol of the polycarbonate resin is 1.8 or greater. This molar ratio is found to provide a method capable of decomposing PC with high reactivity even under mild conditions. No vinyl(co)polymers and/or rubber-modified graft polymers are mentioned.

CN116041934 A discloses a method for separating and recovering PC/ABS blends. After a raw material pretreatment the method comprises the depolymerization of PC/ABS in the presence of an alkaline catalyst, a C₁-C₁₀ alcohol and a solvent to obtain a depolymerization liquid containing depolymerization products, followed by rectification and purification steps to recover bisphenol A, dialkyl (C₁-C₁₀) carbonate and ABS. The ABS is in the liquid phase at the end of the methanolysis reaction due to the presence of the additional solvent. This needs to be removed afterwards in order to obtain a recycled ABS. As solvent 1,4-dioxane, chloroform or chlorobenzene are used which are all solvents which require efforts in handling in a process in industrial scale.

The mentioned documents relate to the depolymerization of polycarbonate compositions or compounds to obtain the polycarbonate building blocks which are then suited for producing new polycarbonates. In case an additional polymeric component is present this component is either separated prior to polycarbonate depolymerization, is present as solid in a slurry, remains as coating on the reactor equipment which needs to be removed in a last step or is actively dissolved by the addition of at least one solvent which needs additional effort in handling.

Only few of the chemical recycling processes known in the literature are currently operated on an industrial scale. This is particularly true for the chemical recycling of compositions and articles (especially end of life articles) not solely comprising polycarbonate but also additional polymeric components as blend partners, additives or coatings. This is mainly due to the lack of process efficiency in terms of limited product yield, the high complexity of processes and/or the limited quality of the reaction products obtained.

It is for example known from the prior art that the methanolysis of PC/ABS blends forms sticky ABS residue in the product mixture. The sticky material complicates subsequent handling and processing, leading to reduced yields of recyclable materials and increased process complexity. This reduces the overall efficiency of the recycling process.

Based on this prior art, it was an object of the present invention to improve at least one known disadvantage. Especially, there was need to develop an efficient and scalable recycling process, preferably a methanolysis for articles comprising polycarbonate and an additional polymeric component. Preferably, it was an object of the present invention to provide a process, preferably a methanolysis which recovers a dihydroxy compound at a high yield and/or good quality and optionally also a vinyl(co)polymer and/or a rubber-modified graft polymer from a polycarbonate blend composition. More preferably, this process, preferably methanolysis should be economically and/or ecologically efficient. In particular this means that the process, preferably methanolysis should need as few resources (including energy and/or (raw) materials such as catalyst, reactants and/or solvents, e. g. methanol and/or dimethyl carbonate) as possible. This is preferably understood that the process should need as few additional resources as possible (e. g. solvents). Moreover, it was preferable that the process works without the use of any materials, e.g. solvents, that require special handling. In addition, it was preferable to have process which avoids the use of halogenated solvents. Moreover, it was preferred to provide a process, preferably a methanolysis for depolymerizing such articles that allows for the efficient depolymerization of polycarbonate into its building blocks (e.g. dihydroxy compounds also referred to as monomers) leading to improved yield and/or purity of the dihydroxy compounds as well as the efficient separation and/or purification of the obtained products. This preferably includes the dihydroxy compounds and all other products. Most preferably, this includes the vinyl(co)polymer and/or a rubber-modified graft polymer contained in the article. This should increase the overall efficiency of the process, preferably methanolysis. Finally, it was an object of the present invention to provide a process, preferably a methanolysis which works at mild process conditions (e. g. low temperature) and still gives high yields, preferably in a short time. Preferably, at least two and most preferably all of the above-mentioned needs were an object of the present invention.

At least one of the above-mentioned objects, preferably all of these objects have been solved by the present invention.

Surprisingly, it was found that the use of the process of a methanolysis provides for effective means to solve at least one, preferably all of the above-mentioned objects. Especially, it was found that the use of the process of a methanolysis of an article comprising polycarbonate and a vinyl(co)polymer and/or a rubber-modified graft polymer, wherein the vinyl(co)polymer and/or a rubber-modified graft polymer is in the liquid state at least at the end of the methanolysis leads to an efficient and scalable recycling process. In particular a dihydroxy compound was obtained at a high yield and/or good quality. Optionally also the vinyl(co)polymer and/or a rubber-modified graft polymer could be recycled. This leads to an economically and/or ecologically efficient process. Due to the requirement that the vinyl(co)polymer and/or a rubber-modified graft polymer is in the liquid state at least at the end of the methanolysis, the required specific ratios of the components are chosen in a way so that as few resources (e. g. energy and/or (raw) materials such as catalyst, reactants and/or solvents, e. g. methanol and/or dimethyl carbonate) as possible are needed. Moreover, this also requires smaller vessels or provides the possibility of recycling larger quantities due to high concentrations (especially when compared to a reaction having the same reaction time).

According to the present invention, the vinyl(co)polymer and/or a rubber-modified graft polymer is in the liquid phase (at least at the end of the methanolysis reaction). This requires the ratios of the components in the reaction mixture of the methanolysis of an article comprising polycarbonate comprising a structural unit derived from a dihydroxy compound and at least one vinyl(co)polymer and/or a rubber-modified graft polymer to be chosen based on the inventive teaching. No additional solvent for the vinyl(co)polymer and/or a rubber-modified graft polymer apart from the components which are used or formed in the inventive process is required. This leads to a simple process where not additional component needs to be removed from the targeted compounds. According to the present invention the formation of a sticky vinyl(co)polymer/rubber-modified graft polymer residue is prevented and at the same time the efficient depolymerization of PC and recovery of its monomers is allowed. More surprisingly, it has been found that the inventive process leads to an increase in the yield of the dihydroxy compound. This increase is preferably seen when compared to the process in which the at least one vinyl(co)polymer and/or a rubber-modified graft polymer is not in the liquid phase in the reaction medium (at the end of the methanolysis reaction). Although for both processes the depolymerization of the PC is assumed to be completed, the yield of the dihydroxy compound is higher for the inventive process. This is especially true when comparing the same effort of washing (e.g. number of washing steps). It was found that the dihydroxy compound sticks to the at least one vinyl(co)polymer and/or a rubber-modified graft polymer. Even when washing this dihydroxy compound cannot be easily removed from the at least one vinyl(co)polymer and/or a rubber-modified graft polymer. This means that a high number of washing steps would be required in order to increase the overall yield of the dihydroxy compound to result in an economically reasonable process. However, when using the inventive process the dihydroxy compound can be recovered (e.g. washed) from the at least one vinyl(co)polymer and/or a rubber-modified graft polymer more easily. This means that less washing steps are needed to get an economically reasonable process. This is especially true when compared to a process where the at least one vinyl(co)polymer and/or a rubber-modified graft polymer is not completely in the liquid phase at the end of the methanolysis reaction. Moreover, this means that a high yield of the dihydroxy compound can be obtained using less solvent for washing and / or less purification steps for recovering the dihydroxy compound resulting in less energy consumption are required. Moreover, the resulting at least one vinyl(co)polymer and/or a rubber-modified graft polymer comprises less dihydroxy compounds especially when referring to the same effort of washing.

Moreover, the inventive process was found to be scalable and at the same time ecologically and/or economically effective. It was found that the at least one vinyl(co)polymer and/or a rubber-modified graft polymer can be precipitated in a way that it is filterable and can be easily removed from the (other) reaction products. This filtration needs low pressures and is very quick.

Finally, the inventive process provides the possibility of using articles of varying particle size and thus is flexible. This is due to the fact that the article or the particles of the article is /are completely in the liquid phase after the methanolysis reaction.

Accordingly, the present invention provides a process for the recovery of
(1) a dihydroxy compound and
(2) optionally a vinyl(co)polymer and/or a rubber-modified graft polymer from an article comprising the steps of
   (i) bringing into contact an article, methanol, at least one catalyst and optionally dimethyl carbonate to form a reaction mixture, wherein the article comprises
      A) polycarbonate comprising a structural unit derived from a dihydroxy compound and
      B) at least one vinyl(co)polymer and/or a rubber-modified graft polymer,
   (ii) performing a methanolysis reaction of the reaction mixture of step (i) yielding at least the dihydroxy compound from component A),
characterized in that component B) is in the liquid phase at least at the end of process step (ii),
wherein the total amount of any further component present in the reaction mixture in step (i) beside the article, methanol, at least one catalyst, optionally dimethyl carbonate, optionally water and optionally any impurity is at most 1 molar equivalent compared to 1 molar equivalent of the structural unit derived from the dihydroxy compound in the polycarbonate of component A).

The inventive process is a process for the recovery of (1) a dihydroxy compound and optionally (2) a vinyl(co)polymer and/or a rubber-modified graft polymer. This means that the inventive process provides a dihydroxy compound as direct product of the process. Sometimes and preferably, the inventive process also provides a vinyl(co)polymer and/or a rubber-modified graft polymer as direct product of the process. Most preferably, the inventive process is a process, wherein the process is for the recovery of (2) the vinyl(co)polymer and/or the rubber-modified graft polymer. This means that (2) is not optional, but two direct products (1) the dihydroxy compound and (2) a vinyl(co)polymer and/or rubber-modified graft polymer are obtained.

The inventive process provides a dihydroxy compound. Sometimes the dihydroxy compound can be referred to as a diol within the context of the present invention. Moreover, the inventive process provides a dihydroxy compound which can be also a mixture of at least two dihydroxy compounds. This means that the inventive process provides at least one dihydroxy compound. It is understood by the skilled person that in case the article comprises at least one copolycarbonate (this means that at least two comonomers are used to obtain the copolycarbonate) more than one dihydroxy compound can be obtained. Preferably, in case the dihydroxy compound is a mixture of at least two dihydroxy compounds, one dihydroxy compound is present to at least 50 %, more preferably at least 60 %, still preferably at least 70 %, still preferably at least 80 %, still preferably at least 90 %, still preferably at least 95 % and most preferably at least 99 % with respect to all dihydroxy compounds.

The dihydroxy compound is obtained by the methanolysis of the component A) of the article. The skilled person knows how dihydroxy compounds can be used in order to obtain a polycarbonate and thus, it is also known to him how a polycarbonate is "derived" from a dihydroxy compound. Typically, those compounds are linked with carbonate groups while each of the two hydrogens is removed and the remaining oxygen is part of the carbonate group. This carbonate group of the polycarbonate is cleaved once again in the methanolysis reaction so that in the end the dihydroxy compound is formed (once again). This dihydroxy compound can be referred to as building block or monomer of the polycarbonate. In this context the term "structural unit" is used according to the invention. It preferably refers to a structural unit derived from a monomer formed by polymerizing a monomer, or a structural unit in which a portion of the structural unit is converted to another structure by processing a polymer. Preferably, the structural unit of the polycarbonate is represented by the following description of the dihydroxy compound, wherein the two hydrogens are removed and which are linked to each other by a carbonate group (cf. above). Accordingly, the skilled person is capable of deriving a structural unit of the polycarbonate when seeing the dihydroxy compound. Moreover, it is understood by the skilled person that the hydroxy compound is organic.

Preferably, the dihydroxy compound comprises, more preferably is a compound selected from the group consisting of formula (1) and (IV) to (X), wherein 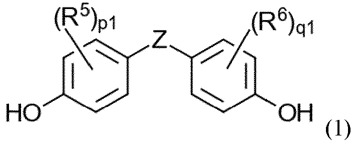
wherein each Z independently represents a single bond, -S(=O)₂-, -C(=O)-, -O-, -S-, -S(=O)-, -CH(CN)-, linear or branched C₁-C₆-alkylene which optionally comprises at least one carbonyl-group, optionally comprises at least one halogen atom and/or optionally is interrupted by at least one heteroatom, C₂-C₁₀-alkylidene which optionally comprises at least one carbon-carbon-double bond, optionally comprises at least one carbonyl-group and/or optionally comprises at least one halogen atom, C₅-C₁₂-cycloalkylene, wherein the cycloaliphatic group is fused to at least one further cycloaliphatic ring, C₅-C₁₅-cycloalkylidene wherein the cycloaliphatic group is optionally fused to at least one cycloaliphatic and /or at least one aromatic ring, C₇-C₂₀-aralkylidene, C₈-C₂₀-aralkylene, C₆-C₁₂-arylene optionally being fused to further aromatic rings which optionally may comprise at least one hetero atom, formula (B1-A), formula (B1-B), formula (B1-C) or formula (B1-D) 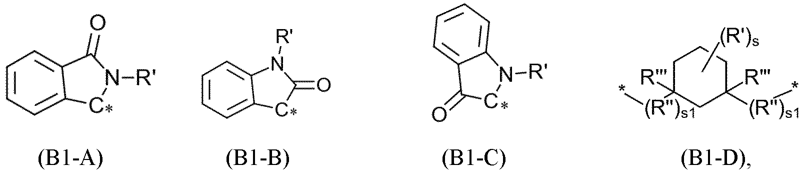
wherein in formulae (B1-A), (B1-B) and (B1-C) each R' independently represents a linear C₁-C₄-alkyl, branched C₃-C₄-alkyl, aralkyl or aryl and the "C*" indicates the quaternary carbon atom which is at the position indicated as "Z" in formula (1), and
wherein in formula (B1-D) each R' independently represents a linear C₁-C₄-alkyl, branched C₃-C₄-alkyl, each R" independently represents a linear C₁-C₄-alkylene, branched C₃-C₄-alkylene, each R‴ independently represents H or linear C₁-C₃-alkyl, s is 0 to 2 and each s1 independently is 0 or 1, and the "*" indicate the bonds which link "Z" to the aromatic rings in formula (1),
each R⁵ and R⁶ independently represents H, C₁-C₁₈-alkyl, C₅-C₆-cycloalkyl, C₁-C₅-alkenyl-, C₁-C₁₈-alkoxy, phenoxy, halogen atom, C₆-C₁₈-aryl or C₇-C₁₈-aralkyl, and
each of p1 and q1 is 1 to 4, 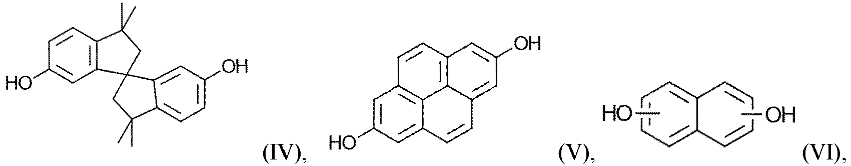 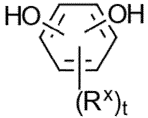 (VII), wherein in formula (VII) each R^{x} independently represents linear or branched C₁-C₆-alkyl, C₁-C₁₂-aryl, C₁-C₈-aralkyl or a halogen atom and t is 0 to 4, 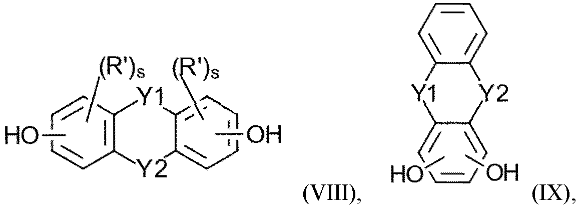 wherein in formulae (VIII) and (IX) each Y1 independently represents oxygen, sulfur or N-R^{y}, wherein R^{y} is H or -CH₃, each Y2 interpedently represents a single bond, oxygen, sulfur or N-R^{y} as defined above and in formula (VIII) each R' independently represents a linear C₁-C₄-alkyl, branched C₃-C₄ alkyl, aralkyl or aryl and s is 0 to 2 and

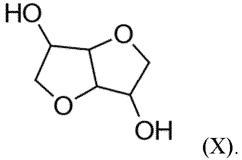

Preferably, the dihydroxy compound is aromatic. More preferably, the aromatic dihydroxy compound is a bisphenol. Most preferably, the dihydroxy compound is a compound of formula (1).

Still preferably, the dihydroxy compound is a compound of formula (1), wherein each Z independently represents a single bond, -S(=O)₂-, -C(=O)-, -O-, -S-, -S(=O)-, linear or branched C₁-C₆-alkylene, C₂-C₁₀-alkylidene which optionally comprises at least one carbon-carbon-double bond, C₅-C₁₅-cycloalkylidene wherein the cycloaliphatic group is optionally fused to at least one cycloaliphatic and /or at least one aromatic ring, C₇-C₁₅-aralkylidene and the C₈-C₁₅-aralkylene of formula (B1-E) 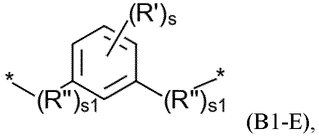
wherein in formula (B1-E) each R' independently represents a linear C₁-C₄-alkyl, branched C₃-C₄ alkyl, each R" independently represents a linear C₁-C₄-alkylene, branched C₃-C₄ alkyl, s is 0 to 2 and each s1 independently is 0 or 1, and the "*" indicate the bonds which link "Z" to the aromatic rings in formula (1),
formula (B1-A), formula (B1-B), formula (B1-C),
each R⁵ and R⁶ independently represents H, C₁-C₁₈-alkyl, C₅-C₆-cycloalkyl, C₁-C₁₈-alkoxy, phenoxy, C₆-C₁₈-aryl or C₇-C₁₈-aralkyl and
each of p1 and q1 is 1 to 4.

More preferably, the dihydroxy compound comprises at least one compound of formula (1), wherein each Z independently represents a single bond, C₂-C₆-alkylidene, C₅-C₁₂-cycloalkylidene wherein the cycloaliphatic group is optionally fused to at least one aromatic ring,
formula (B1-A), formula (B1-B), formula (B1-C),
each R⁵ and R⁶ independently represents H, C₁-C₃-alkyl, C₁-C₁₈-alkoxy,
each of p1 and q1 is 1 to 4.

Still more preferably, the dihydroxy compound is a compound of formulae (1a) or (1b)

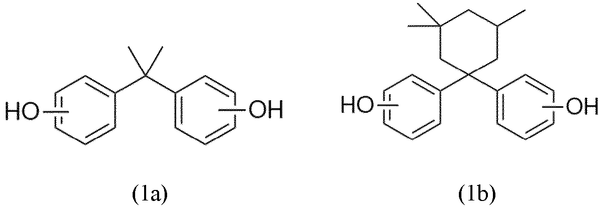

Most preferably, the dihydroxy compound is a compound of formula (1a).

Preferably, in case the dihydroxy compound is a mixture, it is a mixture of at least two compounds of formula (1). In this case it is preferred that at least one compound of formula (1) is present to at least 20 wt.-%, more preferably at least 25 wt.-% and most preferably at least 50 wt.-% with respect to the weight of all compounds of formula (1).

It is also possible that the dihydroxy compound is a compound of formula (1), preferably of formula (1a), in mixture with at least one dihydroxy compound of formula (1c), wherein 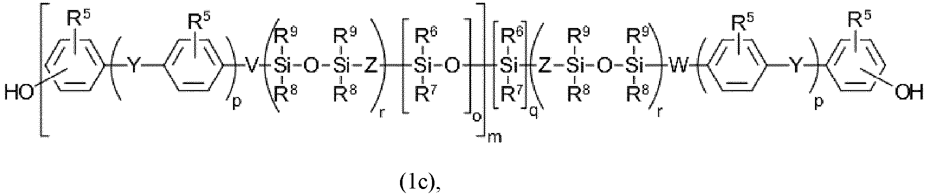 in which
R⁵ represents hydrogen or C₁ to C₄ alkyl, C₁ to C₄ alkoxy, preferably hydrogen or methyl or methoxy, particularly preferably hydrogen,
R⁶, R⁷, R⁸ and R⁹ mutually independently represents C₆ to C₁₂ aryl or C₁ to C₄ alkyl, preferably phenyl or methyl, in particular for methyl,
Y represents a single bond, -SO₂-, -S-, -CO-, -O-, C₁ to C₆ alkylene, C₂ to C₅ alkylidene, C₆ to C₁₂ arylene, which optionally can be condensed to at least one further aromatic ring, which further aromatic ring can comprise at least one hetero atom, or a C₅ to C₆ cycloalkylidene residue, which can be singly or multiply substituted with C₁ to C₄ alkyl, preferably a single bond, -O-, isopropylidene or a C₅ to C₆ cycloalkylidene residue, which can be singly or multiply substituted with C₁ to C₄ alkyl, and in particular isopropylidene,
V represents oxygen, C₂ to C₆ alkylene or C₃ to C₆ alkylidene, preferably oxygen or C₃ alkylene, p, q and r mutually independently each stand 0 or 1,
with the provisio that if q = 0, W represents a single bond, if q = 1 and r = 0, W represents oxygen, C₂ to C₆ alkylene or C₃ to C₆ alkylidene, preferably oxygen or C₃ alkylene,
if q = 1 and r = 1, W and V each independently represent C₂ to C₆ alkylene or C₃ to C₆ alkylidene, preferably C₃ alkylene,
Z represents C₁ to C₆ alkylene, preferably C₂ alkylene,
o represents an average number of repeating units from 10 to 500, preferably 10 to 100 and
m represents an average number of repeating units from 1 to 10, preferably 1 to 6, particularly preferably 1.5 to 5.

Most preferably, the dihydroxy compound is a mixture of at least 20 wt.-%, more preferably at least 25 wt.-% and most preferably at least 50 wt.-% of a compound of formula (1a) and another compound of formula (1) being different from formula (1a), wherein the wt.-% refer to the weight of all dihydroxy compounds which are recovered by the inventive process. It is obvious to the skilled person that in the case where the dihydroxy compound is a mixture of at least two compounds, those compounds could or need to be separated from each other to obtain a respective pure dihydroxy compound.

"Alkyl" in the context of the present invention, for example and if not mentioned differently, refers to an alkane structure of which one hydrogen atom is removed. The "alkyl" of the present invention which can be linear or branched is saturated and therefore, it comprises only single bonds between adjacent carbon atoms. Preferably, alkyl groups according to the present invention comprise methyl, ethyl, n-propyl, isopropyl, n-butyl, sec-butyl, *tert-butyl,* n-pentyl, 1-methylbutyl, 2-methylbutyl, 3-methylbutyl, neopentyl, 1-ethylpropyl, n-hexyl, 1,1-dimethylpropyl, 1,2-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, 1-ethyl-2-methylpropyl and the like. These structures can be limited in choice, in case the invention defines the carbon atoms of an alkyl group in a different manner.

"Alkylene" in the context of the present invention, for example and if not mentioned differently, refers to a bridging alkane structure of which two hydrogen atoms from different carbon atoms are removed. In this context the two carbon atoms of which the two hydrogens atoms are removed can be removed from any carbon atom which is present in the alkane structure. This means that the two carbon atoms can, but not necessarily must be, adjacent to each other. An alkylene structure can be linear or branched and is saturated. In case the alkylene group comprises only one carbon atom, the alkylene group is a methylene group (-CH₂-) which is connected to the rest of the molecule by two single bonds. Preferably, alkylene groups according to the present invention comprise methylene, ethylene, n-propylene, isopropylene, n-butylene, sec-butylene, tert-butylene, n-pentylene, 1-methylbutylene, 2-methylbutylene, 3-methylbutylene, neopentylene, 1-ethylpropylene, n-hexylene, 1,1-dimethylpropylene, 1,2-dimethylpropylene, 1,2-dimethylpropylene, 1-methylpentylene, 2-methylpentylene, 3-methylpentylne, 4-methylpentylene, 1,1-dimethylbutylene, 1,2-dimethylbutylene, 1,3-dimethylbutylene, 2,2-dimethylbutylene, 2,3-dimethylbutylene, 3,3-dimethylbutylene, 1-ethylbutylene, 2-ethylbutylene, 1,1,2-trimethylpropylene, 1,2,2-trimethylpropylene, 1-ethyl-1-methylpropylene, 1-ethyl-2-methylpropylene, 1-ethyl-2-methylpropylene and the like. These structures can be limited in choice, in case the invention defines the carbon atoms of an alkylene group in a different manner. Moreover, the alkylene group according to the present invention optionally comprises at least one carbonyl-group, optionally comprises at least one halogen atom and/or optionally is interrupted by at least one heteroatom. Examples for such alkylene groups are -C(=O)-(CH₂)₄-C(=O)-, -C(=O)-(CH₂)₃-C(=O)-, -C(=O)-(CH₂)₂-C(=O)-, -C(CF₃)₂, -O-(CH₂)₄-O-, -O-(CH₂)₃-O-, -O-(CH₂)₂-O- or the like.

"Alkylidene" in the context of the present invention, for example and if not mentioned differently, refers to a bridging alkane structure of which two hydrogen atoms from the same carbon atom are removed. The alkylidene group optionally comprises at least one carbon-carbon-double bond, optionally comprises at least one carbonyl-group and/or optionally comprises at least one halogen atom. Preferably, alkylidene groups according to the present invention and/or in context with formula (3) comprise CH₂=C*, C(CH₃)₂=C*, isopropylidene, n-propylidene, isoheptylidene, C*(CH₃)(C(=O)CH₃), C(Cl₂)=C*, C(Br₂)=C* or the like, wherein the "C*" indicates the carbon atom which is at the position indicated as "Z" in formula (1).

"Cycloalkylene" in the context of the present invention, for example and if not mentioned differently, refers to a bridging cycloalkane structure of which two hydrogen atoms from different carbon atoms in the ring are removed. In this context the two carbon atoms of which the two hydrogens atoms are removed can be removed from any carbon atom which is present in the cycloalkane structure. This means that the two carbon atoms can, but not necessarily must be, adjacent to each other. According to the present invention the cycloaliphatic group of the cycloalkylene group is fused to at least one further cycloaliphatic ring. Examples of such a cycloalkylene group is the adamantanylene (tricyclo [3.3. 1.1 3,7 ] decanediyl).

"Cycloalkylidene" in the context of the present invention, for example and if not mentioned differently, refers to a bridging cycloalkane structure of which two hydrogen atoms from the same carbon atom in the ring are removed. The cycloaliphatic group of the cycloalkylidene group is optionally fused to at least cycloaliphatic and /or at least one aromatic ring. Examples of such cycloalkylidene groups are cyclopentylidene, cyclohexylidene, 3,3,5-trimethylcyclohexylidene, cyclodecylidene, cyclododecylidene, tetrahydrodicyclopentylidene, 9-fluorenylidene or the like.

"Aralkylidene" in the context of the present invention, for example and if not mentioned differently, refers in each case independently to a bridging straight-chain, cyclic, branched or unbranched alkyl structure of which two hydrogen atoms from the same carbon atom are removed and which is singly, multiply or polysubstituted by aryl radicals. **In** parallel, "aralkylene" refers in each case independently to a bridging straight-chain, cyclic, branched or unbranched alkyl structure of which two hydrogen atoms from the different carbon atoms are removed and which is singly, multiply or polysubstituted by aryl radicals. "Aryl" in the context of the present invention, for example and if not mentioned differently, is a carbocyclic aromatic radical. Examples of "aryl" are phenyl, *o-, p-,* m-tolyl, naphthyl, phenanthryl or anthracenyl. Examples of such aralkylidene groups especially in the context of formula (3) are phenyl-CH*, phenyl-C*(CH₃), naphthyl-CH*, phenyl-C*-phenyl or the like, wherein the "C*" indicates the carbon atom which is at the position indicated as "Z" in formula (1). Examples of such aralkylene groups are m-diisopropylidene phenylene, p-diisopropylidene phenylene.

"Alkoxy" in the context of the invention, for example and if not mentioned differently, refers to a linear, cyclic or branched alkyl group singularly bonded to oxygen (-OR). Preferably, alkoxy groups according to the present invention have 1 to 6 carbon atoms and, thus, comprise methoxy, ethoxy, n-propoxy, isopropoxy, n-butoxy, sec-butoxy, tert-butoxy, n-pentoxy, 1-methylbutoxy, 2-methylbutoxy, 3-methylbutoxy, neopentoxy, 1-ethylpropoxy, cyclohexoxy, cyclopentoxy, n-hexoxy, 1,1-dimethylpropoxy, 1,2-dimethylpropoxy, 1,2-dimethylpropoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy or 1-ethyl-2-methylpropoxy. These structures can be limited in choice, in case the invention defines the carbon atoms of an alkoxy group in a different manner.

A "halogen atom" in the context of the invention, if not mentioned differently, refers to fluorine (F), chlorine (Cl), bromine (Br), or iodine (I). Preferably, a halogen atom is F, Cl, Br or I, more preferably it is Cl or Br.

Based on the above-given definitions the skilled person knows how to understand further definitions which are not explicitly defined in the passage above.

### Component A)

As already described above, the skilled person is capable of identifying the connection between the polycarbonate which is used as component A) and the dihydroxy compound as recovered according to the inventive process. In the context of the present invention the term "polycarbonate" refers to a polymer that contains multiple carbonate groups. These carbonate groups are incorporated into the polymer backbone. This means that a polycarbonate preferably has repeating units of the form ...-(R-O-(C=O)-O)-...

According to the present invention in the process an article is used which comprises a polycarbonate comprising a structural unit derived from a dihydroxy compound as component A) (e. g. HO-R-OH, wherein the R represents the R as given for the above-shown repeating units of the polycarbonate). This polycarbonate is degraded by the process of the present invention to give the dihydroxy compound. Accordingly, the skilled person can understand the structure of the polycarbonate based on the structure as given for the dihydroxy compound. As example, the skilled person knows the structure of bisphenol A based polycarbonate.

This means that "polycarbonates" in the context of the present invention generally comprise other structures besides the carbonate base structures. It is also possible that they comprise even other structures besides the carbonate base structures and the structural units which are derived from the dihydroxy compound. For example, typically a polycarbonate comprises a chain terminator. Moreover, this can be especially true in case the article which comprises component A) is post-industrial waste or post-consumer waste, also known as end-of-life (EoL) material. Those materials might have structures which can be called "defective structures" due to the lifecycle of the polycarbonate. However, such "defective structures" can also be present in the virgin polycarbonate. Their amount could be higher than the amount in virgin polycarbonate. Polycarbonate chains are known to degrade under harsh conditions and/or UV light leading to rearrangement structures etc. Those structures are known to the skilled person. The presence of such structures diverging from the pure base structure does not depart from the scope of the present invention. Especially when referring to such EoL materials the exact structure of the polycarbonate might be unknown. However, the skilled person is able to determine whether the polycarbonate is derived from a dihydroxy compound (and in most cases also which exact compound). This is especially true as most of those materials comprise polycarbonate derived from bisphenol A.

According to the present invention, the exact structure and /or molecular weight of the polycarbonate in the article is preferably not considered. The chemical nature of the dihydroxy compound is determined. By this the chemical nature of the structural unit derived from the dihydroxy compound in the polycarbonate of component A) can be determined, too. This can be done by methods known to the skilled person. Preferably, the chemical nature of the polycarbonate can be determined by IR-spectroscopy and/or NMR-spectroscopy. It is also possible to firstly depolymerize the polymer (e. g. by saponification as described below) and to conduct a gas chromatography. Those methods are known to the skilled person. For example, when using IR spectroscopy, a KBr pellet of the article can be analyzed and compared to a calibration. By this the polycarbonate content can be determined. It is also possible to determine the weight of polycarbonate in the article by saponification of the article and thus, the polycarbonate. This leads to the full degradation of the polymer chain to its monomers. This method might be more appropriate in case the IR spectrum of the article itself has significant overlaps of signals.

For example, the article could be subjected to a total hydrolysis to form the corresponding degradation products. This may be accomplished for example as follows: The article is hydrolyzed under reflux by means of sodium methoxide (for example in dichloromethane). The corresponding solution is acidified (for example by HCl) and concentrated to dryness. The drying residue is dissolved in acetonitrile and the dihydroxy compounds are determined for example by means of HPLC with UV detection.

From the results, the skilled person is capable of calculating the molar equivalents of the structural unit derived from the dihydroxy compound in the polycarbonate of component A).

Polycarbonates according to component A) which are suitable according to the invention are known from the literature or can be produced by processes known from the literature (for production of polycarbonates see by way of example Schnell, "Chemistry and Physics of Polycarbonates", Interscience Publishers, 1964 and also DE-AS (German Published Specification) 1 495 626, DE-A 2 232 877, DE-A 2 703 376, DE-A 2 714 544, DE-A 3 000 610, DE-A 3 832 396).

Aromatic polycarbonates are produced by way of example by reaction of diphenols with carbonyl halides, preferably phosgene and/or with aromatic diacyl dihalides, preferably dihalides of benzenedicarboxylic acids, by the interfacial process, optionally using chain terminators, for example monophenols, and optionally using trifunctional or more than trifunctional branching agents, for example triphenols or tetraphenols. Production via a melt polymerization process by reaction of diphenols with, for example, diphenyl carbonate is likewise possible.

Preferably, the polycarbonate of component A) comprises at least one structural unit of formula (3) to (10) 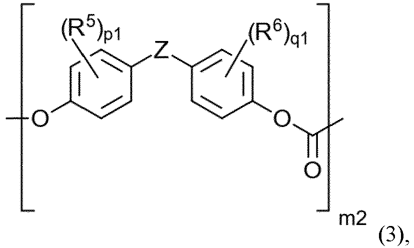
wherein each Z each R^{5,} R⁶ and each of p1 and q1 have the meanings as given above for formula (1) and m2 represents the average number of repeating units, 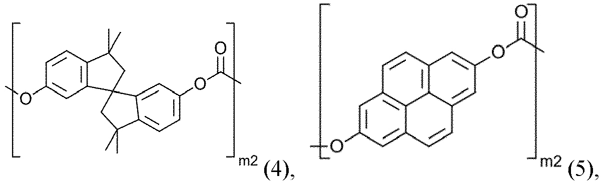 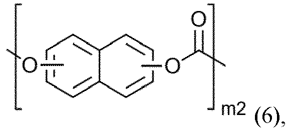
wherein in formula (4) to (6) m2 represents the average number of repeating units; 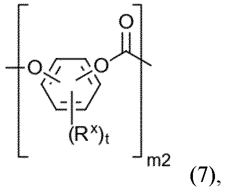
wherein each R^{x} independently represents linear or branched C₁-C₆-alkyl, C₁-C₁₂-aryl, C₁-C₈-aralkyl or a halogen atom, t is 0 to 4 and m2 represents the average number of repeating units; 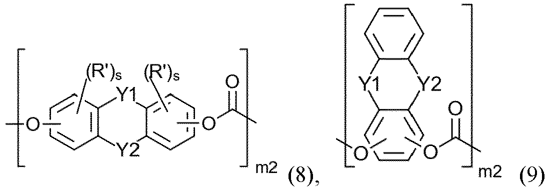
wherein in formula (8) and (9) each Y1 independently represents oxygen, sulfur or N-R^{y}, wherein R^{y} is H or -CH₃, each Y2 interpedently represents a single bond, oxygen, sulfur or N-R^{y} as defined above and m2 represents the average number of repeating units and in formula (8) each R' independently represents a linear C₁-C₄-alkyl, branched C₃-C₄ alkyl, aralkyl or aryl and s is 0 to 2; 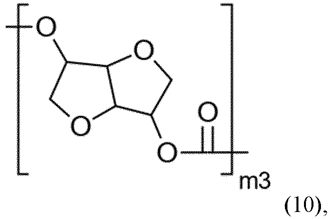
wherein m3 represents the average number of repeating units.

Moreover, all preferred meanings and combinations of preferred meanings with respect to above-given formula (1) apply. In accordance with the above-given meanings for formula (1), the polycarbonate of component A) most preferably comprises structural units represented by formula (2a) and/or (2b) 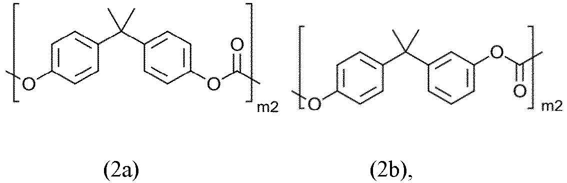 wherein m2 represents the average number of repeating units, preferably 6 to 60.

Moreover, the polycarbonate of component A) preferably comprises at least one structural unit of formula (3) above, most preferably of formula (2a), and a structure of formula (2c) 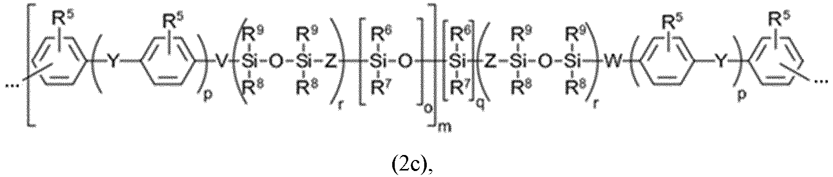 in which
R⁵ represents hydrogen or C₁ to C₄ alkyl, C₁ to C₄ alkoxy, preferably hydrogen or methyl or methoxy, particularly preferably hydrogen,
R⁶, R⁷, R⁸ and R⁹ mutually independently represents C₆ to C₁₂ aryl or C₁ to C₄ alkyl, preferably phenyl or methyl, in particular for methyl,
Y represents a single bond, -SO₂-, -S-, -CO-, -O-, C₁ to C₆ alkylene, C₂ to C₅ alkylidene, C₆ to C₁₂ arylene, which optionally can be condensed to at least one further aromatic ring, which further aromatic ring can comprise at least one hetero atom, or a C₅ to C₆ cycloalkylidene residue, which can be singly or multiply substituted with C₁ to C₄ alkyl, preferably a single bond, -O-, isopropylidene or a C₅ to C₆ cycloalkylidene residue, which can be singly or multiply substituted with C₁ to C₄ alkyl, and in particular isopropylidene,
V represents oxygen, C₂ to C₆ alkylene or C₃ to C₆ alkylidene, preferably oxygen or C₃ alkylene,
p, q and r mutually independently each stand 0 or 1,
with the provisio that if q = 0, W represents a single bond, if q = 1 and r = 0, W represents oxygen, C₂ to C₆ alkylene or C₃ to C₆ alkylidene, preferably oxygen or C₃ alkylene,
if q = 1 and r = 1, W and V each independently represent C₂ to C₆ alkylene or C₃ to C₆ alkylidene, preferably C₃ alkylene,
Z represents C₁ to C₆ alkylene, preferably C₂ alkylene,
o represents an average number of repeating units from 10 to 500, preferably 10 to 100,
m represents an average number of repeating units from 1 to 10, preferably 1 to 6, particularly preferably 1.5 to 5 and "..." represents the sites at which the structure of formula (2c) is incorporated into the polycarbonate.

The term "average number of repeating units" is known by the skilled person. The skilled person knows how to determine this parameter. Typically, it is determined by using a GPC method. Formulae (2a) and / or (2b) recite "m2" as number of repeating units. Preferably, m2 is 6 to 60, more preferably it is 10 to 55, even more preferably it is 20 to 50, still more preferably it is 25 to 45 and most preferably it is 30 to 40. Preferably, it is determined using the GPC method as outlined in the context of the present invention. Accordingly, according to the present invention it is preferred that the polycarbonate of component A) has a weight average molecular weight of 15000 g/mol to 40000 g/mol, more preferably 16000 g/mol to 34000 g/mol, still more preferably 17000 g/mol to 33000 g/mol and most preferably 19000 g/mol to 32000 g/mol as determined *via* size exclusion chromatography. Preferably, this size exclusion chromatography is calibrated using a bisphenol A polycarbonate and dichloromethane as eluent. Typically, linear polycarbonate (obtained from BPA and phosgene) having a known molecular weight distribution of PSS Polymer Standards Service GmbH, Deutschland can be used for the calibration. Most preferably, the calibration is performed according to method 2301-0257502-09D method (from 2009 in German) from the company Currenta GmbH & Co. OHG, Leverkusen. The eluent is dichloromethane. Preferably, a combination of columns based on crosslinked styrene divinylbenzene resins is used. Still preferably, the diameter of the analytical columns is 7,5 mm, length of 300 mm. The particle size of the column material is preferably 3 µm to 20 µm. The concentration of the solution is preferably 0.2 wt.-%. The flow rate is preferably 1.0 ml/min. The temperature of the solution is preferably 30 °C. Preferably, a UV- and/or RI-detection is used.

According to the present invention component A) can comprise more than one polycarbonate. This means that component A) can be a mixture of at least two polycarbonates. However, especially when referring to EoL materials this cannot easily be determined. As outlined above, the knowledge of such a fact is not detrimental in order to carry out the process of the present invention.

### Component B)

According to the present invention, the article comprises at least one vinyl(co)polymer and/or a rubber-modified graft polymer as component B). According to the present invention the vinyl(co)polymer can be also referred to as "rubber-free vinyl(co)polymer". This especially helps to distinguish the rubber-modified graft polymer and the vinyl(co)polymer which is rubber free.

Preferably, the rubber-modified graft polymer comprises
B.1 5 to 95% by weight, preferably 20 to 92% by weight, in particular 30 to 91% by weight, based on the graft polymer, of at least one vinyl monomer on
B.2 95 to 5% by weight, preferably 80 to 8% by weight, in particular 70 to 9% by weight, based on the graft polymer, of one or more rubber-elastic graft substrates having glass transition temperatures < -10°C, more preferably < -40°C, particularly preferably < -70°C, determined by dynamic scanning calorimetry (DSC) according to DIN EN 61006 in the version of 2004 at a heating rate of 10 K/min with determination of Tg as the midpoint temperature (tangent method).

The graft substrate B.2 preferably has a median particle size (D50) of 0.05 to 10.00 µm, preferably of 0.1 to 5.0 µm, and particularly preferably of 0.1 to 1.5 µm.

The median particle size D50 is the diameter with 50 % by weight of the particles above it and 50 % by weight below it. Unless expressly stated otherwise in the present invention it is determined for all components by means of ultracentrifuge measurement (e. g. W. Scholtan, H. Lange, Kolloid, Z. und Z. Polymere [Polymers] 250 (1972), 782-1796).

The monomers B.1 are preferably mixtures of
- B.1.1: 65 to 85 % by weight, particularly preferably 70 to 80 % by weight, more preferably 74 to 78 % by weight, in each case based on the sum of B.1.1 and B.1.2, of vinylaromatics and/or ring-substituted vinylaromatics (such as styrene, α-methylstyrene, p-methylstyrene, p-chlorostyrene) and/or (C₁-C₂₀)-alkyl (meth)acrylates, preferably (C₁-C₈)-alkyl (meth)acrylates, such as methyl methacrylate, ethyl methacrylate, and
- B.1.2: 15 to 35 % by weight, particularly preferably 20 to 30 % by weight, more preferably 22 to 26 % by weight, in each case based on the sum of B.1.1 and B.1.2, of vinyl cyanides (unsaturated nitriles such as acrylonitrile and methacrylonitrile) and/or (C₁-C₂₀)-alkyl (meth)acrylates, preferably (C₁-C₈)-alkyl (meth)acrylates, such as methyl methacrylate, n-butyl acrylate, t-butyl acrylate, and/or derivates (such as anhydrides and imides) of unsaturated carboxylic acids, for example maleic anhydride.

Preferred monomers B.1.1 are selected from at least one of the monomers styrene, α-methylstyrene and methyl methacrylate; preferred monomers B.1.2 are selected from at least one of the monomers acrylonitrile, maleic anhydride and methyl methacrylate. Particularly preferred monomers are B.1.1 styrene and B.1.2 acrylonitrile. Alternatively preferred monomers are B.1.1 methyl methacrylate and B.1.2 methyl methacrylate.

Suitable graft substrates B.2 of the graft polymers include for example diene rubbers, EP(D)M rubbers, i.e. those based on ethylene/propylene and optionally diene, acrylate, polyurethane, silicone, chloroprene, ethylene/vinyl acetate and also acrylate-silicone composite rubbers.

Preferred graft substrates B.2 are diene rubbers, preferably comprising butadiene or copolymers of dienes, preferably comprising butadiene, and further copolymerizable vinyl monomers (e.g. according to B.1.1 and B.1.2) or mixtures of one or more of the above-mentioned components.

A particularly preferred graft substrate B.2 is pure polybutadiene rubber. In a further preferred embodiment B.2 is styrene-butadiene rubber, particularly preferably styrene-butadiene block copolymer rubber.

Alternatively preferred graft substrates B.2 are acrylate rubbers, preferably selected from polymers of alkyl acrylates, optionally with up to 40 % by weight, based on B.2, of other polymerizable, ethylenically unsaturated monomers. Preferred alkyl acrylates include C₁ to C₈ alkyl esters, especially methyl, ethyl, butyl, n-octyl and 2-ethylhexyl esters; halogenated alkyl esters, preferably halogenated C₁ to C₈ alkyl esters, such as chloroethyl acrylate, and mixtures of these monomers. Preferred 'other' polymerizable, ethylenically unsaturated monomers which, in addition to the alkyl acrylates, can optionally be used to produce the graft substrate B.2 are, for example, acrylonitrile, styrene, *-methyl styrene, acrylamides, vinyl C₁ to C₆ alkyl ether, methyl methacrylate, butadiene.

The gel fraction of the graft substrate B.2 is at least 30 % by weight, preferably at least 40 % by weight, in particular at least 60 % by weight, in each case based on B.2 and measured as insoluble fraction in toluene.

The gel content of the graft substrate B.2/of the graft polymers in component B is determined at 25 °C in a suitable solvent as content insoluble in these solvents (M. Hoffmann, H. Krömer, R. Kuhn, Polymeranalytik I und II, Georg Thieme-Verlag, Stuttgart 1977).

Most preferred as rubber-modified graft polymer is acrylonitrile-butadiene-styrene (ABS).

The graft copolymers in component B) are produced by free-radical polymerization, for example by emulsion, suspension, solution or bulk polymerization. Mixtures of graft polymers produced in different processes may also be used as component B. The graft polymers in component B are preferably produced by emulsion or bulk polymerization.

Suitable graft polymers produced in the emulsion polymerization process are for example ABS polymers produced in the emulsion polymerization process by redox initiation with an initiator system composed of organic hydroperoxide and ascorbic acid according to US-P 4 937 285.

Further suitable graft polymers produced in the emulsion polymerization process are MBS modifiers having a core-shell structure.

Suitable polymers according to component B) prepared by bulk polymerization are described for example in DE-OS 2 035 390 (=US PS 3 644 574) or in DE-OS 2 248 242 (=GB-PS 1 409 275), or in Ullmanns, Enzyklopädie der Technischen Chemie, Vol. 19 (1980), p. 280 et seq.

The rubber-modified graft polymer may comprise free, i.e. not chemically bonded to the rubber substrate and not included in the rubber particles, vinyl(co)polymer. This vinyl(co)polymer can be also referred to as rubber-free vinyl(co)polymer (cf. above). This means that typically, when component B) comprises a rubber-modified graft polymer in addition it also comprises at least one (rubber-free) vinyl(co)polymer. This means component B) preferably is at least one vinyl(co)polymer and a rubber-modified graft polymer. The vinyl(co)polymer preferably comprises structural units derived from the vinyl monomers according to B.1 above. Preferably, the vinyl(co)polymer comprises structural units derived from at least one vinyl monomer selected from the group consisting of vinylaromatics, ring-substituted vinylaromatics, (C1-C8)-alkyl (meth)acrylates, vinyl cyanides and unsaturated carboxylic acids. More preferably, the vinyl(co)polymer comprises structural units derived from at least one vinyl monomer selected from the group consisting of vinylaromatics, (C1-C8)-alkyl (meth)acrylates and vinyl cyanides. It is understood that also combinations of the mentioned vinyl monomers are encompassed.

Such vinyl(co)polymer may be formed in the rubber-modified graft polymer during the polymerization of the graft polymers owing to the method of production (the grafting on the graft substrate is not necessarily complete) or may else be polymerized and admixed with the rubber-modified graft polymer separately. It is likewise possible for a portion of the free vinyl(co)polymer in the rubber-modified graft polymer to originate from the graft polymer itself owing to the method of production and for another portion to be polymerized and admixed with the rubber-modified graft polymer separately.

Preferably, this free vinyl(co)polymer has in the rubber-modified graft polymer a weight-average molecular weight M_{w} of 30 to 250 kg/mol, preferably of 70 to 200 kg/mol, in particular of 90 to 180 kg/mol (measured by gel permeation chromatography (GPC) in tetrahydrofuran against a polystyrene standard). Preferably and in particular preferably in the context of the given weight-average molecular weight above the vinyl(co)polymer is styrene acrylonitrile resin (also known as SAN).

In the context of the present invention, the weight average molecular weight Mw of the free vinyl(co)polymer in the rubber-modified graft polymer is measured by gel permeation chromatography (GPC) in tetrahydrofuran against a polystyrene standard.

According to the present invention, component B) can be also at least one vinyl(co)polymer with or without the additional presence of a rubber-modified graft polymer. Thus, this vinyl(co)polymer is rubber-free. Therefore, according to the present invention the vinyl(co)polymer is sometimes also referred to as rubber-free vinyl(co)polymer. This especially serves to distinguish between the at least one vinyl(co)polymer and the rubber-modified graft polymer. A vinyl(co)polymer is understood to be rubber-free if it is neither formed during the polymerization of a rubber-modified graft polymer nor polymerized and then admixed with a rubber-modified graft polymer. This means that the vinyl(co)polymer is polymerized and may then be admixed with a polycarbonate comprising a structural unit derived from a dihydroxy compound and optionally one or more polymer additives or polymeric components, without any rubber-modified graft polymer being present in the composition. It is also possible that the separately polymerized vinyl(co)polymer may be admixed with a polycarbonate and optionally rubber-modified graft polymer. Preferably, component B) consists of at least one vinyl(co)polymer.

The rubber-free vinyl(co)polymer preferably comprises structural units derived from at least one vinyl monomer B.1 as defined above. More preferably, the vinyl(co)polymer is styrene-acrylonitrile copolymer, polyalkyl (meth)acrylate or alkyl (meth)acrylate copolymer. Particularly preferably, the vinyl(co)polymer is styrene-acrylonitrile copolymer and/or poly(methyl methacrylate).

Here and elsewhere according to the invention, the term "(meth)acrylate" refers to acrylates or methacrylates.

The (rubber-free) styrene-acrylonitrile copolymer preferably has a weight-average molecular weight Mw of 30 to 250 kg/mol, preferably of 70 to 200 kg/mol, in particular of 90 to 180 kg/mol (measured by gel permeation chromatography (GPC) in tetrahydrofuran against a polystyrene standard).

It is understood that polyalkyl (meth)acrylate may be a polyalkyl (meth)acrylate or a mixture of different polyalkyl (meth)acrylates. The polyalkyl (meth)acrylate is preferably a linear polymer.

The polyalkyl (meth)acrylate preferably has a weight-average molecular weight M_{w} of 50,000 to 2,000,000 g/mol, preferably of 80,000 bis 300,000 g/mol, further preferably of 100,000 bis 200,000 g/mol (measured by gel permeation chromatography (GPC) in tetrahydrofuran under PMMA calibration).

The polyalkyl (meth)acrylate can also be a mixture of two or more polyalkyl (meth)acrylates, one of which can also be of low molecular weight. The mentioned weight-average molecular weight then refers accordingly to the total polyalkyl (meth)acrylate mixture. At least one of these can be low molecular weight and have a weight-average molecular weight of 1,000 to 70,000 g/mol, most preferably 5,000 to 60,000 g/mol. The low-molecular weight polyalkyl (meth)acrylate preferably has a proportion of 2 to 20 wt.%, in particular 5 to 10 wt.%, based on the total weight of the polyalkyl (meth)acrylate. This improves the processability with the polycarbonate. The previously stated weight-average molecular weight of 50,000 to 2,000,000 g/mol, preferably of 80,000 to 300,000 g/mol, further preferably 100,000 to 200,000 g/mol, refers to the total polyalkyl (meth)acrylate contained in the composition according to the invention.

The polyalkyl (meth)acrylate preferably comprises
a) 52.0 to 100.0 wt.% of alkyl methacrylate repeating units with 1 to 20, preferably 1 to 12, more preferably 1 to 8, particularly preferably 1 to 4, carbon atoms in the alkyl radical, in particular methyl methacrylate,
b) 0 to 48.0 wt.% of alkyl acrylate repeating units containing 1 to 20, preferably 1 to 12, more preferably 1 to 8, particularly preferably up to 4, carbon atoms in the alkyl radical, in particular methyl acrylate, and
c) 0 to 8.0wt.% of styrenic repeating units of the general formula (VII) 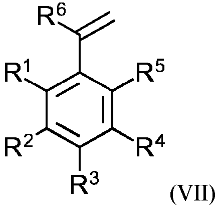 where R¹ to R⁵ independently of one another are hydrogen, halogen, C₁- to C₆-alkyl or C₂-to C₆-alkenyl and R⁶ is hydrogen or C₁- to C₆-alkyl
where the amounts in wt.% of a, b and c add up to 100.0 wt.%, the total weight of the polyalkyl (meth)acrylate.

Very preferably, the polyalkyl (meth)acrylate comprises at least 60.0 wt.%, even more preferably at least 75.0 wt.%, in particular at least 85.0 wt.% methyl methacrylate repeating units.

Most preferably, the polyalkyl (meth)acrylate comprises 90.0 to 99.0 wt.% of methyl methacrylate and 1.0 to 10.0 wt.% of methyl acrylate, in each case based on the total weight of the polyalkyl (meth)acrylate.

In the context of the present invention, the polyalkyl (meth)acrylate is particularly preferred to be poly(methyl methacrylate). Preferably, the polyalkyl (meth)acrylate has a weight average molecular weight of 55 000 g/mol to 85 000 g/mol, more preferably of 60 000 g/mol to 80 000 g/mol (as determined via GPC in THF using a polystyrene calibration).

As described above with respect to component A), in EoL materials the chemical nature and/or the amount of component B) might be unknown. In order to determine the amount of component B) in the article, it is possible to depolymerize the article (e. g. saponify it as described above) and to determine the amount of the residue. It is also possible to analyze this residue to determine the chemical nature of component B).

### Article

According to the present invention an "article" is used from which the dihydroxy compound and optionally the vinyl(co)polymer and/or a rubber-modified graft polymer is or are recovered. The article comprises components A) and B) as described above. Typically, the article comprises a blend of at least component A) and B). However, it might be also possible that the article comprises component A) and component B) which are spatially separated, for example at least two layers, one layer of component A) and one layer of component B). Moreover, the article may comprise further components and/or layers in addition to component A) and B). Therefore, the article can comprise a composition comprising at least component A) and B). In this case the article may comprise further layers such as coatings or the article is a multilayer article. Finally, the article may be a mixture of different articles which comprises components A) and B) (e. g. a shredded article comprising component A) is mixed with a shredded article comprising B) to result in a mixed article comprising component A) and B). This may be encompassed when using the expression "the article comprises components A) and B)".

An "article" according to the present invention preferably means an article produced by processing a thermoplastic composition comprising at least component A) and B) into a (shaped or molded) part. Processing methods are known to the skilled person and include any plasticization method commonly used for polycarbonate, e.g. granulation, injection molding, (co)extrusion, blow molding, thermoforming. The article can have many different shapes and sizes, e.g. granules, pellets, injection-molded parts, sheets, films. Preferably, the article originates from post-industrial waste or post-consumer waste. This means that preferably, it is an end-of-life (EoL) material.

According to the present invention sometimes the term "polycarbonate blend composition" or "polycarbonate composition" is used. This refers to a composition comprising at least component A) and B).

The article according to the present invention comprises A) polycarbonate comprising a structural unit derived from a dihydroxy compound and B) at least one vinyl(co)polymer and/or a rubber-modified graft polymer. The polycarbonate preferably comprises structural units derived from the dihydroxy compound of formula (1), more preferably from the dihydroxy compound of formulae (1a) or (1b), most preferably from the dihydroxy compound of formula (1a).

Preferably and most preferably in this context, component B) comprises as vinyl(co)polymer styrene-acrylonitrile copolymer, polyalkyl (meth)acrylate and/or alkyl (meth)acrylate copolymer and/or as rubber-modified graft polymer acrylonitrile-butadiene-styrene.

The article may optionally comprise one or more polymer additives or polymeric components C). Here, it is preferable that the composition forming the article comprising A) and B) comprise one or more polymer additives or polymeric components C). It is understood that in case the polymeric components C) are present, they are no polycarbonate, no vinyl(co)polymers and/or no rubber-modified graft polymers. Preferably, component C) is selected from the group consisting of flame retardants, anti-drip agents, flame retardant synergists, smoke inhibitors, lubricants and demolding agents, nucleating agents, antistatic agents, conductivity additives, stabilizers (e.g. heat stabilizers, hydrolysis and heat aging stabilizers, UV stabilizers and also transesterification inhibitors), flow promoters, phase compatibilizers, further polymeric constituents other than components A) and B) (for example functional blend partners), fillers and reinforcing agents, light diffusing agents, dyes and pigments. The article may optionally comprise one or more layers selected from the group consisting of coating layers, primer layers and co-extruded layers. This is especially the case when the article comprises a composition comprising at least component A) and B).

Preferably, component C) is selected from the group consisting of thermal stabilizers, antioxidants, UV absorbers, lubricants and mold release agents.

In the context of the present invention component C), if present, does not necessarily need to be in the liquid phase at least of inventive process step (ii). This obviously depends on the chemical nature of component C).

Preferably, the article consists of at least 75 wt.-%, more preferably at least 80 wt.-%, still preferably at least 85 wt.-%, still preferably at least 90 wt.-%, still preferably at least 95 wt.-% and most preferably at least 98 wt.-% of component A), B) and optionally C), based on the weight of the whole article.

In the context of the present invention, "article" can mean a single article or a mixture of articles. A mixture of articles may include articles of the same origin, e.g. type of application and/or type of material or material mixture, or articles of multiple different origins. Even if articles of the same origin are present, they may still differ in terms of material composition and/or quality.

It may be advantageous to reduce the particle size of the article by processes known to the skilled person such as shredding, crushing, cutting or milling prior to (bringing the article into contact with methanol, a catalyst and optionally dimethyl carbonate according to) step (i) of the process according to the present invention. A smaller particle size may increase the surface and also the depolymerization rate. Preferably, the average particle size of the article is below 1 cm, more preferably below 0.5 cm. This particle size preferably refers to the largest dimension of the shredded article. Within the context of the present invention all measures to reduce the size of the collected molded parts are preferably referred to as shredding and the articles obtained after shredding are referred to as shredded articles. Most preferably, inventive process step (i) comprises bringing into contact at least a shredded article, methanol, at least one catalyst and optionally dimethyl carbonate to form a reaction mixture.

### Catalyst

The methanolysis reaction is carried out in the presence of at least one catalyst. If sometimes it is referred to as "a catalyst" or "the catalyst", this is understood as "at least one catalyst". Thus, it is also feasible to employ mixtures of different catalysts.

Preferably, the at least one catalyst is a transesterification catalyst. Preferably, the catalyst is a base.

The catalyst is preferably selected from the group consisting of alkali metal or earth alkali metal hydroxides, alkali metal or earth alkali metal carbonates, alkali metal or earth alkali metal alkoxides, alkali metal or earth alkali metal oxides, alkylamines, and pyridine.

The alkali metal or earth alkali metal hydroxide is preferably selected from the group consisting of lithium hydroxide, sodium hydroxide, calcium hydroxide and potassium hydroxide.

The alkali metal or earth alkali metal carbonate is preferably selected from the group consisting of sodium carbonate, sodium hydrogen carbonate, potassium hydrogen carbonate and potassium carbonate.

The alkali metal or earth alkali metal alkoxide is preferably selected from the group consisting of lithium methoxide, sodium methoxide, calcium methoxide and sodium ethoxide.

The alkali metal or earth alkali metal oxide is preferably selected from the group consisting of sodium oxide, lithium oxide, calcium oxide and potassium oxide. The catalyst is more preferably an alkali metal hydroxide. Even more preferably, the catalyst is sodium hydroxide. This catalyst was found to provide sufficiently high depolymerization rates while being economically affordable.

The catalyst can be added as such or in solution. For example, sodium hydroxide can be added in water and/or methanol. Most preferably, the catalyst sodium hydroxide is added in water.

Preferably, the catalyst is used in an amount of 0.01 to 10 wt.-%, preferably 0.05 to 5 wt.-%, still preferably 0.1 to 1 wt.-% and most preferably 0.2 to 0.5 wt.-% with respect to component A).

### Process step (i)

According to the present invention in process step (i) at least an article, methanol, at least one catalyst and optionally dimethyl carbonate are brought into contact to form a reaction mixture. The article and the catalyst are described above.

In process step (i) the total amount of any further component present in the reaction mixture beside the article, methanol, at least one catalyst, optionally dimethyl carbonate, optionally water and optionally any impurity is at most 1 molar equivalent compared to 1 molar equivalent of the structural unit derived from the dihydroxy compound in the polycarbonate of component A). This preferably means that the content of any other component is low. It was found according to the present invention that these components are able to transfer component B) into the liquid phase at least at the end of step (ii). This means that no other component is needed in step (i). However, it is obvious to the skilled person that other components such as e. g. impurities from the methanol, catalyst(s), the article and optionally the dimethyl carbonate might be present.

According to the present invention, the term "bringing into contact" is used. This is intended to encompass both embodiments in which one component/mixture etc. is added to another component/mixture etc., as well as the reverse order. How such contact between components and/or mixtures is established is known to those skilled in the art. This can be achieved, for example, by adding a component and/or mixture, introducing a component and/or mixture and afterwards adding another component and/or mixture, mixing a component and/or mixture, dripping in a component and/or mixture, etc. It is apparent to those skilled in the art that the term "adding," as occasionally used in the invention, can be understood as synonymous with "bringing into contact." The "bringing into contact in step (i) is preferably carried out with introduction of mixing energy. This may be carried out by methods known to those skilled in the art. It is known that increasing surface renewal influences the rate of methanolysis.

Moreover, according to the present invention it may not be possible to clearly distinguish between process step (i) and (ii). The presence of the at least one catalyst in process step (i) may immediately initiate the methanolysis reaction. However, this does not depart from the present invention. This means that "before step (ii)" preferably means "in step (1)".

Most preferably, in inventive step (i) at least a shredded article, methanol, at least one catalyst and optionally dimethyl carbonate are brought into contact. Methanol is required to perform a methanolysis reaction. Thus, the lowest possible amount of methanol required in process step (ii) is predetermined by the amount of component A) being present or rather the amount of structural units derived from the dihydroxy compound being present in the polycarbonate of component A). However, it is highly preferred to use higher amounts than this lowest possible amount of methanol in order for the reaction mixture to have a manageable viscosity. Moreover, the highest possible amount of methanol in the inventive process depends on the other conditions, but can be determined by the skilled person. As will be explained later, methanol is an anti-solvent for component B). Thus, too much methanol might lead to a precipitation of component B) or prevent the transfer to the liquid phase of component B). Still this depends on the type of component B), the amount of dimethyl carbonate present (as this is a solvent for component B), the amount of the dihydroxy compound present (as this is a solvent for component B) and therefore, also on the ratio of component A) to component B), because this determines how much dihydroxy compound will be formed with respect to the amount of component B) present.

Moreover, the expression that dimethyl carbonate is "optionally" brought into contact with the article, methanol and the catalyst is understood by the skilled person. Dimethyl carbonate is a solvent for component B). This means that according to the present invention the expression "component B) is dissolved" or "in a dissolved state" is used. However, depending on the chemical nature of component B), an emulsion can be formed optionally in the presence of a dissolved component B) (the latter can be the case if component B) comprises at least two components). This is the reason why sometimes it is described that component B) is "in the liquid state". Preferably, this means that component B) is present in the mixture as obtained after step (ii) as an emulsion and/or in a dissolved state. The skilled person knows that an emulsion is a finely distributed mixture of two normally immiscible liquids without visible separation. Most preferably, the emulsion is stable. This preferably refers to a mixture of at least two immiscible liquids where one liquid is dispersed in the other in the form of fine droplets, and this dispersion remains uniform over time without phase separation. According to the present invention this is encompassed when referring to a "solvent". Especially in case when component B) is a mixture of ABS and SAN, it was found that SAN seems to be at least partly dissolved after step (ii) and the ABS is present as emulsion.

As dimethyl carbonate is a solvent for component B) it has the ability to dissolve and/or form an emulsion with component B). The methanolysis reaction leads to the formation of dimethyl carbonate. Depending on the amount of component B) in the article (and maybe other conditions as described according to the present invention) different amounts of dimethyl carbonate are needed to support the dissolution and/or formation of an emulsion of component B). As said above, the dihydroxy compound to be formed according to the present invention is a solvent for component B), too. For example, this means in case a high amount of component A) is present in the article (and a respectively low amount of component B)), it is possible that the dimethyl carbonate and the dihydroxy compound that are generated due to the methanolysis reaction suffice in order to transfer component B) into the liquid phase at least at the end of process step (ii). In case a high amount of component B) is present in the article (and a respectively low amount of component A), it might be required to add dimethyl carbonate already in process step (i) in order to ensure that component B) is in the liquid phase at the end of process step (ii). The skilled person is capable of conducting a few experiments and then calculate and predict whether an additional amount of dimethyl carbonate is needed in process step (i) or not.

Preferably, in inventive step (i) no further solvent, especially no further solvent for component B) is present. More preferably, in inventive step (i) no 1,4-dioxane, methylenchloride, tetrahydrofuran, N-methyl pyrrolidone, dimethyl sulfoxide, 1,3-dimethyl-2-imidazolidinone, chloroform and/or chlorobenzene is present. Still more preferably, in inventive step (i) no 1,4-dioxane, chloroform and/or chlorobenzene is present. According to the present invention water can be present in process step (i) and/or (ii). This can be intentionally added, for example when the catalyst is firstly dissolved in water and then used in process step (i). Moreover, it is also possible that water is introduced in process step (i) by residual moisture in the article.

Moreover, in case any solvent (e. g. methanol and/or dimethyl carbonate) is recycled from the inventive process to process step (i), there might be some water in process step (i). Preferably, the amount of water in process step (i) is less than 3 wt.-% with respect to the reaction mixture of step (i). According to the present invention, it was found that water is an anti-solvent for component B). Moreover, it is an even stronger anti-solvent for component B) than methanol. This means that the presence of water influences the solubility product of component B). Accordingly, if water is present in process step (i) and/or (ii), higher amounts of solvents for component B) (i. e. dimethyl carbonate and/or dihydroxy compound) are required (wherein "higher" refers to the same parameters, but without the presence of water). The skilled person is capable of conducting a few experiments and then calculate and predict whether due to the presence of water an additional amount of dimethyl carbonate is needed in process step (i) or not or whether the amount of component B) needs to be lowered in the system (e. g. by diluting the article with another article having a higher amount of component A)). It is highly recommended to limit the amount of water present in the inventive process. However, as described, this sometimes cannot be fully excluded. More preferably, the amount of water in process step (i) is less than 2 wt.-%, most preferably less than 1 wt.-% with respect to the reaction mixture of step (i). The term "less than" preferably encompasses 0 wt.-%. Most preferably the term "less than" means > 0 wt.-%.

According to the present invention, the total amount of any further component present in the reaction mixture in step (i) beside the article, methanol, at least one catalyst, optionally dimethyl carbonate, optionally water and optionally any impurity is at most 1 molar equivalent compared to 1 molar equivalent, preferably at most 0.8 molar equivalent compared to 1 molar equivalent, still preferably at most 0.5 molar equivalent compared to 1 molar equivalent, still preferably at most 0.1 molar equivalent compared to 1 molar equivalent, still preferably at most 0.05 molar equivalent compared to 1 molar equivalent, most preferably at most 0.01 molar equivalent compared to 1 molar equivalent of the structural unit derived from the dihydroxy compound in the polycarbonate of component A). The expression "any further component" especially includes solvents or other substances that have an impact on the solubility / capability of component B) to be in the liquid phase.

According to the present invention, it is preferred that the impurity being optionally present in step (i) is selected from
(a) any impurity being introduced into step (i) by the article, methanol, the at least one catalyst, optionally the dimethyl carbonate and / or optionally the water and
(b) any impurity being introduced into step (i) by methanol, dimethyl carbonate and / or water, wherein the methanol, dimethyl carbonate and / or water is recovered from process step (ii), optionally purified and reused in process step (i).

The impurity of option (a) originates form the compounds itself. **In** contrast to option (b) this refers to "fresh" components. These are used for the first time in process step (i). For example, water could be present as residual moisture in the article, methanol is contaminated with traces of impurities and / or the catalyst comprises impurities.

The impurity of option (b) is originates from the recycling of components. Such a recycling is preferably described below. It is possible to reuse the methanol, dimethyl carbonate and/or water which is obtained after process step (ii) once again in process step (i). Therefore, it needs to be separated from the other components of process step (ii). It can be optionally further purified (cf. below). When reusing methanol, dimethyl carbonate and/or water any impurity of the fresh methanol, dimethyl carbonate and/or water might still be present in the reused methanol, dimethyl carbonate and/or water. Moreover, the methanol, dimethyl carbonate and/or water might comprise at least one impurity and / or side product of the methanolysis reaction. This means that this impurity and / or side product can be either present alone or in addition to any impurity of the fresh methanol, dimethyl carbonate and/or water. It is understood that "impurity" means "at least one impurity" as there might be several ones.

Preferably, the inventive process is characterized in that the impurity being introduced into step (i) by option (b) originates from any impurity and / or side product of the methanolysis reaction of step (ii) of the article or is the dihydroxy compound which is produced in step (ii). Such mentioned side product or side products could originate from component B) and / or component C). The presence of these components might lead to unwanted reaction (i. e. no methanolysis reaction). Once again, these impurities might be present in methanol, dimethyl carbonate and/or water in case this is reused once again in step (i).

If at least one of methanol, dimethyl carbonate and water (if present at all) from step (ii) is reused in step (i) once again, it is possible that option (a) and option (b) are both true. For example, if methanol is reused, it might comprise impurities and/or side products of option (b). In addition, a part of the methanol and /or dimethyl carbonate in process step (i) might be fresh methanol and / or dimethyl carbonate and might introduce impurities of option (a).

Preferably, the process of the present invention is characterized in that the impurity which is optionally present in step (i) is at most 15 wt.-%, more preferably 13 wt.-%, still more preferably 10 wt.-% and most preferably 5 wt.-%.

Preferably, the reaction mixture of step (i) essentially comprises the article, methanol, at least one catalyst, optionally dimethyl carbonate, optionally water and optionally any impurity. Most preferably, the reaction mixture of step (i) consists of the article, methanol, at least one catalyst, optionally dimethyl carbonate, optionally water and optionally any impurity. Wherein the "impurity" is preferably the impurity as defined above. It is preferred that any composition of the reaction mixture of step (i) is the same as the composition of the reaction mixture before step (ii). Moreover, it has an impact on the composition at the end of step (ii).

According to the present invention it is advantageous to have as few components in the reaction mixture of step (i) as possible. This leads to an easier work up after methanolysis. Moreover, substances that are not that easy to handle (e. g. 1,4-dioxane, chloroform and/or chlorobenzene) and/or are halogenated can be avoided.

### Process step (ii)

In process step (ii) a methanolysis reaction is performed. This process is known to the skilled person. According to the present invention "methanolysis" preferably refers to a process that depolymerizes polycarbonate to produce dihydroxy compounds and dimethyl carbonate by using methanol. In principle, this reaction is a transesterification. Methanol as alcohol transesterifies the carbonate group which is bound to the structural unit derived from the dihydroxy compound resulting in a methanol carbonate bond (and if performed twice in dimethyl carbonate) and a degraded polymer chain with a hydroxy end group (and if performed twice at a structural unit of the polycarbonate it results in the dihydroxy compound). By way of example, the following scheme shows the theoretical methanolysis reaction of bisphenol A based polycarbonate (e.g. the reaction which is aimed at, possible side reactions are not shown):

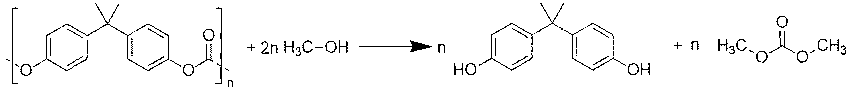

Therefore, the methanolysis reaction is capable of depolymerizing polycarbonate chains to yield at least the dihydroxy compound. The skilled person is aware that a copolycarbonate which comprises more than one structural unit yields more than one dihydroxy compound. Moreover, it was found that small amounts of water can be tolerated in this reaction and do not significantly influence the reaction as shown above.

Process step (ii) is preferably carried out in a temperature range of the reaction mixture from 50°C to 130 °C, preferably 60 °C to 120 °C, even more preferably 90 °C to 115 °C. Preferably, process step (ii) is conducted at a temperature which is above the glass transition temperature of component B). It was found that the overall reaction velocity significantly increases above the glass transition temperature of component B).

Preferably, the term "in a liquid state" at the end of inventive process step (ii) when referring to component B) refers to the temperature in which process step (ii) is conducted. More preferably, the term "in a liquid state" at the end of inventive process step (ii) when referring to component B) refers even to room temperature. This means that even when the mixture obtained by process step (ii) is cooled to room temperature, component B) preferably is still in the liquid phase. So, it is preferred that component B) is in the liquid phase at least at the end of process step (ii), wherein the term "liquid phase" refers to room temperature. This does not necessarily mean that the mixture as obtained by step (ii) needs to be cooled to room temperature according to the present invention. It rather refers to the condition which would be true if the mixture was cooled to room temperature.

The expression "at least at the end of process step (ii)" preferably means that in case the conversion of component A) is at least 90 %, more preferably at least 95 %, most preferably at least 98 % component B) is in the liquid phase. That does not exclude that even earlier in the methanolysis reaction of step (ii) component B) is fully in the liquid phase. This depends on the composition of the reaction mixture (as describe according to the present invention) and the temperature. Most preferably, "at the end of process step (ii)" no further dimethyl carbonate and/or dihydroxy compound is formed.

Process step (ii) is preferably carried out in a pressure range from 1 bara to 15 bara, preferably 1.5 bara to 13 bara and most preferably 2 bara to 11 bara. Most preferably, process step (ii) is carried out in the above-given temperature range of the reaction mixture and at the above-given pressure range.

Process step (ii) is preferably carried out for 2 minutes to 20 hours, more preferably for 3 minutes to 5 hours, most preferred for 5 minutes to 1 hours. It is further preferred that the depolymerization of the polycarbonate is at least 95 % complete, further preferred at least 98 % complete, more preferred at least 99%, most preferred at least 99.5 %. A less complete depolymerization of polycarbonate leads to higher amounts of residual oligocarbonates in the product mixture at the end of process step (ii). This not only reduces the yield of the dihydroxy compound which can be recovered but also requires the removal of such oligocarbonates before recovering the dihydroxy compound and optionally the vinyl(co)polymer and/or a rubber-modified graft polymer. It is therefore preferred that the polycarbonate conversion in the process according to the invention is as high as possible. It is understood that this actual yield is not the same as the theoretical yield of 100 % which underlies the calculation of conditions (a) to (c). This means that even if the methanolysis reaction is not carried out to 100 % conversion, the assumption that the methanolysis reaction of component A) is theoretically completed to 100 % for the calculation of conditions (a) to (c) still apply.

Preferably, process step (i) and/or process step (ii) are conducted under inert atmosphere (e. g. using nitrogen or argon).

Preferably, at the end of step (ii) the conversion of the methanolysis reaction is at least 80 %, more preferably at least 90 %, still preferably at least 95 % and most preferably at least 98 %. It is beneficial to have a high conversion in order for the process to be economically and/or ecologically efficient.

The skilled person is capable of finding conditions, e. g. determine the optimal composition of process step (i) (and thus step (ii)), where the feature that component B) is in the liquid phase at the end of step (ii) is met. Especially taking into account the teaching of the present examples he is able to adapt any amount of methanol, dimethyl carbonate and /or component A).

In case an article comprising end-of-life material or any other article of unknown composition is used in step (i) of the present invention, the composition of the article has to be analyzed with respect to the kind and amount of component A) and component B) prior to process step (i) (cf. above). The skilled person knows suitable methods for such analysis, for example infrared spectroscopy as explained above. The amount of polycarbonate present in an article is the basis for calculating the weight of the hydroxy compound to be produced by the methanolysis reaction. If the exact amount of component B) cannot be determined, preferably the maximum possible amount thereof is considered. The maximum possible amount of component B) is preferably determined by the weight difference of the article and the amount of polycarbonate present therein, for example if the amount of polycarbonate in the article has been analyzed to be 60 wt.-%, the maximum possible amount of component B) is 40 wt.-%. Using this assumption, it can be guaranteed that component B) is indeed in a liquid state at the end of process step (ii).

Surprisingly, it was found that the reaction velocity of the methanolysis is faster in case component B) is in the liquid phase during and/or at the end of the methanolysis (when compared to the same reaction where component B) not in the liquid phase). Moreover and most surprisingly, it was found that the yield of the hydroxy compound is higher. As can be seen from the experiments, the amount of hydroxy compound which is readily separated from component B) after step (ii) is high. It is even higher when compared to a process step (ii) in which component B) is not in a liquid phase (e. g. as slurry or as coating at the reactor wall). This already leads to a higher yield of the hydroxy compound, because the loss of the hydroxy compound which sticks to component B) is smaller. Moreover, the examples surprisingly show that a high amount of hydroxy compound which is stick to component B) can be removed. This increases the yield of the hydroxy compound even further. That is to say that the loss of hydroxy compound is smaller. This is especially the case when compared to a process in which component B) is not in the liquid phase during and/or at the end of the methanolysis reaction. In the end this means that the inventive process provides for a high yield of hydroxy compound. Preferably, the inventive process provides a yield of the hydroxy compound of at least 75 %, more preferably at least 80 %, still preferably of at least 85 %, still preferably of at least 90 %, still preferably of at least 95 % and most preferably of at least 98 %. For the skilled person it is clear that the number of washing steps of component B) contributes to this yield. However, it is not economically and/or ecologically reasonable to have a high number of washing steps. At the same time the purity of component B) which is recovered is very high. This means that the effort for any work up of component B) in order to recycle this component is low.

According to the present invention it was found that due to the reaction of the methanolysis the solution conditions of process step (ii) continuously change. With the aim to bring and/or keep component B) in a liquid state, this needs to be considered. The terms "dissolve" and/or "in solution" and/or "in a liquid state" preferably mean that, when filtering the liquid in which component B) is present, no solid can be separated off using customary filter methods and/or that the liquid phase remains uniform over time without phase separation. Moreover, it is preferred that the term "in a liquid state" refers to room temperature (cf. above). Dimethyl carbonate is a good solvent for component B), whereas methanol is an anti-solvent. This also means that depending on the chemical nature of component B) dimethyl carbonate can form an emulsion with component B), whereas methanol does not form an emulsion with component B). The term "anti-solvent" is understood by the skilled person. It preferably refers to a liquid which reduces the solubility product of component B) in a given (highly concentrated) solution of component B). This preferably means that it is capable of precipitating component B) from a (highly concentrated) solution of component B). The generated dihydroxy compound is a good solvent for component B), too. During the methanolysis reaction the amounts and ratios of dimethyl carbonate, methanol and the dihydroxy compound change. Methanol is consumed, whereas dimethyl carbonate and the dihydroxy compound is generated. Accordingly, the conditions constantly vary. The amount of component B) in the initial article is decisive whether dimethyl carbonate needs to be added from or whether the generated dimethyl carbonate is sufficient. In the case where additional dimethyl carbonate is needed to guarantee that component B) is in the liquid state at the end of the methanolysis reaction, it can be added at the very beginning of step (ii) and/or during process step (ii). Moreover, it is preferred that in the inventive process as few resources as possible are used in order to guarantee an economically and ecologically advantageous process. This means for example that even if higher amounts of dimethyl carbonate guaranteed that component B) is in the liquid phase at least at the end of process step (ii), it would not be economically and /or ecologically beneficial to add a high amount of dimethyl carbonate. This is especially true if this high amount of dimethyl carbonate is not needed to ensure the presence of component B) in the liquid phase.

A high amount of methanol could lower the viscosity of the mixture as obtained after inventive step (ii). However, it could lead to precipitation of component B) and will need to be removed afterwards from the dihydroxy compound. Therefore, in order to keep the process economic and ecological, the amount of methanol should be as low as possible (in the boundaries of the methanolysis reaction).

Preferably, the viscosity after performing step (ii) is in the range of not more than 1 000 mPa*s, more preferably of not more than 500 mPa*s, still more preferably of not more than 300 mPa*s, still more preferably of not more than 100 mPa*s and most preferably of not more than 50 mPa*s. Preferably, these viscosity numbers refer to the temperatures used in step (ii). Methods how to determine the viscosity are known to the skilled person. It is apparent that the viscosities are greater than 0 mPa*s and that the lower the viscosity, the easier the workup after step (ii) will be. These indicated viscosities are manageable and at the same time the amount of methanol is not too high so that the process is still economically and ecologically advantageous. Preferably, the viscosity after preforming step (ii) is adjusted by the addition of methanol in step (i), step (ii) or at the end of step (ii).

According to the present invention it was found that the amount of methanol and dimethyl carbonate have more influence on the dissolution of component B) than the dihydroxy compound. It is preferable to keep the amount of dimethyl carbonate added to the reaction mixture of step (i) at a minimum. This increases the production efficiency. Additional amounts of dimethyl carbonate lead to an increased volume of the reaction mixture and thus the amount of energy required for heating and mixing, as well as to increased efforts necessary for subsequent workup, e.g. separation of solvents, separation of component B) and side products from the desired products. Furthermore, it is unfavorable to add too large amounts of dimethyl carbonate to the reaction mixture of step (i) as this means that the amount of dimethyl carbonate generated during the methanolysis reaction of step (ii) has to be low. In this case the content of component A) in the article with respect to the content of component B), both present in the reaction mixture of step (i), is low. Thus, the amount of dihydroxy compound to be recovered is also low. This has a negative impact on the absolute amount of the dihydroxy compound to be obtained based on the mass of the whole article making it less economical attractive. It is therefore preferable to add 0 to less than 1.8 mol of dimethyl carbonate relative to 1 mol of dihydroxy compound.

As said above, the it is also possible to decrease the amount of component B) in step (i) byadjusting the ratio of component A) : component B) in the reaction mixture of step (i). As component A) is depolymerized during the methanolysis reaction in step (ii), dimethyl carbonate is generated. The skilled person can calculate the amount of dimethyl carbonate which is produced under the assumption that the methanolysis reaction of component A) is completed to 100 %. The ratio of component A) : component B) can for example be adjusted by adding component A) to the reaction mixture of step (i) or by mixing articles with different component A) : component B) ratios. By this the total amount of dimethyl carbonate which is needed can also be influenced. It is preferred to adjust the ratio of component A) : component B) by increasing the amount of component A) rather than to increase the amount of dimethyl carbonate.

It is preferred that the amount of component A) in the article is at least 25 wt.-%, more preferably at least 30 wt.-% and most preferably 36 wt.-%. A high amount of component A) in the article increases the overall yield and thus production efficiency of the recovery of the hydroxy compound. More preferably in order to get an economically attractive process, the amount of component A) in the article is at least 40 wt.-%, more preferably at least 50 wt.-% and most preferably 60 wt.-%. These amounts of component A) preferably relate to the total content of component A) in case a mixture of articles is used.

### Further process steps

It is preferred that the process according to the present invention further comprises the steps of
(iii) precipitating component B) from the product as obtained after performing step (ii) to obtain a suspension comprising a solid fraction comprising the vinyl(co)polymer and/or the rubber-modified graft polymer and a liquid fraction comprising the dihydroxy compound and (iv) separating the solid fraction and the liquid fraction of the suspension of step (iii).

The precipitation of component B) can be achieved by any means known to the skilled person. Preferably, the precipitation of component B) is achieved by lowering the solubility of component B) in the mixture as obtained after performing step (ii), e.g. by lowering the temperature and/or changing the solubility conditions. This can be done either by addition of an anti-solvent or by removal of a solvent. As also defined before "anti-solvent" preferably means a solvent in which a compound is less soluble. If an anti-solvent is used for the precipitation of component B), the anti-solvent preferably is a substance which is present at the end of step (ii) of the inventive process. Most preferably, the anti-solvent is methanol and/or water. Preferably, the precipitation of component B) is achieved by the addition of methanol. This is advantageous, because methanol is already present in the system. This means that the further work-up is advantageous, because no further components need to be removed. It is also advantageous for recovering the methanol. For example, the methanol could be recycled and used in process step (i). Using methanol as anti-solvent in process step (iii) does not add any further components which might have a negative impact on the recyclability of the methanol. However, as indicated before, when recycling methanol the amount some side products and /or water could increase in process step (i).

According to the present invention it was found that due to the inventive process a precipitation of component B) is possible. This results in a processable suspension. Preferably, this means that the suspension can be pumped and/or removed from the reactor. In comparison to this if component B) is not in the liquid phase at the end of process step (ii) it sticks to the reactor wall and is difficult to be removed from the reactor.

The separation of the solid fraction and the liquid fraction of the suspension (process step (iv)) can be done by any solid/liquid separation process known to the skilled person, e.g. filtration, sedimentation, decantation and/or centrifugation. Preferably, the solid fraction and the liquid fraction are separated using filtration. It was found that when using the process of the present invention precipitated component B) can be easily filtered off and/or washed. This is due to the fact that component B) forms a fine, powdery residue in form of a suspension of low viscosity. In this context the term "low viscosity" preferably refers to a viscosity of < 20 mPas. It is known to the skilled person that filtration of mixtures with viscosities of > 20 mPas is technically not reasonable. In contrast to this, if component B) is not in the liquid phase after process step (ii) (e. g. comparative examples), it forms a sticky, phase-separated mass of high viscosity (> 20 mPas). Due its high viscosity this mass is not filterable, it cannot be washed with any washing agent and thus, the desired products which might be still present in this mass cannot be removed (e. g. the dihydroxy compound cannot be washed off component B)). Optionally, the solid fraction can be washed after the separation step in order to remove any remaining anti-solvent or other compounds present at the end of step (ii) of the inventive process which are not component (B), e.g. dihydroxy compound, methanol, catalyst and dimethyl carbonate, water, residual oligocarbonates. Preferably, the solid fraction is washed after the separation step. This additional step of washing can be used in order to increase the overall yield of the dihydroxy compound, because by washing component B), remaining dihydroxy compound can be recovered. For the washing for example methanol and/or water can be used.

Particular preferably, the inventive process is a process wherein a vinyl(co)polymer and/or the rubber-modified graft polymer is recovered and which additionally comprises the steps of isolating the vinyl(co)polymer and/or the rubber-modified graft polymer. Most preferably, in this case, the inventive process comprises inventive process steps (iii) and (iv) and a step of isolating the vinyl(co)polymer and/or the rubber-modified graft polymer.

It was found that when using the inventive process the recyclability of the vinyl(co)polymer and/or the rubber-modified graft polymer is very good. As explained above, it was found that component B) can be easily separated from any other component that are soluble in the washing liquid present after process step (ii). It has a good quality. It was found that it has a low residual content of dihydroxy compound. Moreover, it has a low residual content of potentially other low molecular, organic compounds beside the dihydroxy compound (such as for example chain terminator and / or potential additives of the article such as component C)). Preferably, the recovered vinyl(co)polymer and/or the recovered rubber-modified graft polymer has a low residual BPA content of preferably less than 5 wt.-%.

The liquid fraction obtained after the separation step (iv) can undergo any liquid/liquid separation process known to the skilled person, e.g. distillation or extraction, in order to separate at least methanol and dimethyl carbonate from the dihydroxy compound. Preferably, a mixture comprising at least methanol and dimethyl carbonate and the dihydroxy compound is obtained. It is also possible that a stream comprising methanol and dimethyl carbonate and a stream comprising the dihydroxy compound are obtained.

The stream comprising methanol and dimethyl carbonate may be reused in the methanolysis of polycarbonates. Preferably, the stream comprising methanol and dimethyl carbonate can be reused in inventive process step (i). This might require exact knowledge of the composition of the stream which is, however, routine experimentation for the skilled person. Alternatively, the stream comprising methanol and dimethyl carbonate can be separated into a methanol-rich stream and a dimethyl carbonate-rich stream. Here it is preferred that the methanol-rich stream contains more than 75 wt.-%, more preferably more than 85 wt.-% and most preferably more than 90 wt.-% methanol. Moreover, it is preferred that the dimethyl carbonate-rich stream contains more than 30 wt.-%, more preferably more than 60 wt.-% and most preferably more than 95 wt.-% dimethyl carbonate. The methanol-rich stream can be reused to depolymerize polycarbonates. Preferably, the methanol-rich stream is reused in inventive process step (i). Furthermore, the methanol- and/or dimethyl carbonate-rich stream can be purified before use in further reactions. This can be done by processes known to the skilled person.

The present invention preferably provides a process for the recovery of (1) the dihydroxy compound, further comprising the step of
(v) isolating the dihydroxy compound.

As said above, preferably after step (iv) a liquid fraction is obtained which comprises methanol, dimethyl carbonate and the dihydroxy compound. It is also possible that such a liquid fraction can be obtained from inventive step (ii) other than by inventive steps (iii) and (iv).

The dihydroxy compound can be isolated from such a liquid fraction comprising methanol, dimethyl carbonate and the dihydroxy compound, preferably after inventive process step (iv) by a liquid/liquid separation process. Such processes are known to the skilled person. The purity of the dihydroxy compound can be increased by methods known to the skilled person. The dihydroxy compound can be further purified by one or more extraction and crystallization process steps which is a known method known to the skilled person. Preferably, the recovered dihydroxy compound has a purity of at least 90 %, more preferably of at least 95 % and most preferably of at least 98 %.

Moreover, using the inventive process it was found that the yield of the dihydroxy compound is very high, especially in addition to the high purity. Preferably, the yield of the dihydroxy compound is at least 70 %, more preferably at least 80 %, most preferably at least 90 %. This yield can be preferably obtained in combination with the above-given purity of the dihydroxy compound. For the skilled person it is understood that the percentage of the yield is based on the amount of component A) which theoretically decomposes to 100 % to the dihydroxy compound. For the calculation of the yield preferably all assumptions as with respect to conditions (a) to (c) are applied which relate to component A). For example for calculating the yield, the presence of chain terminator groups and /or branching points are ignored.

Due to the use of the inventive process such good quality (e. g. purity) and / or high yield of the dihydroxy compound is obtained using a small effort for washing and / or few purification steps. Thus, low energy consumption is required.

In a particular preferred embodiment the present invention provides a process comprising inventive steps (i) and (ii) and further comprises the steps of
(iii) precipitating component B) to obtain a suspension comprising a solid fraction comprising a vinyl(co)polymer and/or a rubber-modified graft polymer and a liquid fraction comprising the dihydroxy compound,
(iv) separating the solid fraction from the liquid fraction of the suspension as obtained in step (iii) and optionally washing the solid fraction to recover the vinyl(co)polymer and/or the rubber-modified graft polymer,
(v) removing at least methanol and dimethyl carbonate from the liquid fraction of step (iii) preferably by distillation or evaporation to recover the dihydroxy compound.

Step (v) might further comprise at least one, preferably all of the following steps:
(v-1) obtaining a mixture comprising the dihydroxy compound after the distillation or evaporation in step (v),
(v-2) dissolving the mixture of step (v-1) preferably with toluene to obtain a solution of the hydroxy compound,
(v-3) crystallizing the dihydroxy compound from the solution of the hydroxy compound, preferably followed by filtrating the dihydroxy compound,
(v-4) drying of the dihydroxy compound.

In a further aspect of the present invention a process is provided for the production of a polymer comprising the step of polymerizing the dihydroxy compound recovered in the process according to the present invention. It is understood that this inventive process can comprise any of the described steps even in preferred embodiments or in combinations of preferred embodiments.

Accordingly, the present invention further provides a process for the production of a polymer comprising the step of polymerizing a dihydroxy compound, wherein the hydroxy compound is produced by a process comprising the steps of
(i) bringing into contact an article, methanol, at least one catalyst and optionally dimethyl carbonate to form a reaction mixture, wherein the article comprises
   A) polycarbonate comprising a structural unit derived from a dihydroxy compound and
   B) at least one vinyl(co)polymer and/or a rubber-modified graft polymer,
(ii) performing a methanolysis reaction of the reaction mixture of step (i) yielding at least the dihydroxy compound from component A),
characterized in that component B) is in the liquid phase at least at the end of process step (ii),
wherein the total amount of any further component present in the reaction mixture in step (i) beside the article, methanol, at least one catalyst, optionally dimethyl carbonate, optionally water and optionally any impurity is at most 1 molar equivalent compared to 1 molar equivalent of the structural unit derived from the dihydroxy compound in the polycarbonate of component A).

Preferably, the polymer is polycarbonate or an epoxy resin. Most preferable the dihydroxy compound is bisphenol A.

Furthermore, the invention provides a process for the production of a polymer blend comprising the step of blending the vinyl(co)polymer and/or a rubber-modified graft polymer recovered in the inventive process with a further polymer. It is understood that this inventive process can comprise any of the described steps even in preferred embodiments or in combinations of preferred embodiments.

Accordingly, the present invention provides a process for the production of a polymer blend comprising the step of blending the vinyl(co)polymer and/or a rubber-modified graft polymer with a further polymer, wherein the vinyl(co)polymer and/or a rubber-modified graft polymer is recovered using the steps of
(i) bringing into contact an article, methanol, at least one catalyst and optionally dimethyl carbonate to form a reaction mixture, wherein the article comprises
   A) polycarbonate comprising a structural unit derived from a dihydroxy compound and
   B) at least one vinyl(co)polymer and/or a rubber-modified graft polymer,
(ii) performing a methanolysis reaction of the reaction mixture of step (i) yielding at least the dihydroxy compound from component A),
characterized in that component B) is in the liquid phase at least at the end of process step (ii),
wherein the total amount of any further component present in the reaction mixture in step (i) beside the article, methanol, at least one catalyst, optionally dimethyl carbonate, optionally water and optionally any impurity is at most 1 molar equivalent compared to 1 molar equivalent of the structural unit derived from the dihydroxy compound in the polycarbonate of component A).

Preferably, the "further polymer" is a thermoplastic polymer, most preferably polycarbonate. Still preferably, the vinyl(co)polymer and/or a rubber-modified graft polymer is ABS, SAN or PMMA.

According to the present invention the term "comprising" refers to compositions or process steps which at least contain the indicated features. Moreover, "comprising" preferably revers to "consisting essentially of". Most preferably, the term "comprising" means "consisting of.

### Examples

### Analytics:

### Nuclear Magnetic Resonance Spectroscopy (1H NMR):

The NMR measurements were performed on a Spinsolve 80 Ultra device from magritek at 80 MHz at 300 K. The spectra were normalized to the corresponding resonances of the solvent: 1H: δ = 2.50 ppm (DMSO-d6) or δ = 3.31 ppm (MeOH-d4). The chemical shift was given in ppm. For quantification, a known amount of pyrazine (1H: δ = 8.5 ppm (s)) was added as an internal standard. The NMR spectra were evaluated with the MestReNova software.

### High-Performance Liquid Chromatography (HPLC):

The quantification of Bisphenol A (BPA) in Acrylonitrile Butadiene Styrene (ABS) was performed using an Agilent Technologies 1260 Infinity II HPLC system. The system included a G7112B pump, G7129A sampler, G7116A thermostat, and G7117A UV detector. A Restek UHPLC PreColumn filter (0.2 µm) was used as a prefilter. The chromatographic separation was achieved on an Agilent ZORBAX Eclipse Plus C18 column (150 mm × 4.6 mm, 5 µm particle size, 95 Å pore size) at 35 °C. The pressure limit was set to ≥ 400 bar. The mobile phases consisted of ultrapure water (Solvent A) and 100 % methanol (Solvent B). The injection volume was 5 µL, and the sampler temperature was maintained at 35 °C. Detection was performed using a diode array detector (DAD) with signals at 254 nm, 278 nm, and 240 nm. The spectrum was recorded from 190 to 400 nm with a data collection rate of 2.5 Hz. Sample preparation involved weighing approximately 50 mg of ABS into a 20 mL screw-cap vial, adding 10 mL of acetonitrile, and sonicating for 10 minutes. The solution was then diluted with water to precipitate the ABS. The diluted solutions were filtered with 0.2 µm PTFE filters before injection. Quantification was performed using an external calibration with seven BPA standards (5.3 to 33.6 ppm). The calibration standards were prepared in the same solvent composition as the sample extracts.

### Gas Chromatography (GC):

The analysis of isolated Bisphenol-A (BPA), was conducted using an Agilent 6890 gas chromatograph equipped with a flame ionization detector (FID), an automatic injector, and AGILENT Chemstation software (version A.06.03). The chromatographic separation was achieved using a WCOT quartz glass column (Chrompack:nr7749, now Agilent) with CP-Sil 5CB as the stationary phase, an internal diameter of 0.32 mm, a film thickness of 0.4 µm, and a length of 50 meters. For the analysis of BPA in its solid form, 10 mg (±1 mg) of the dried product was placed into a GC vial, followed by the addition of 150 µL of silylation reagent (N-Methyl-N-trimethylsilyltrifluoracetamid (MSTFA)). The mixture was heated on the GC detector's heating block for 10 minutes until fully dissolved, then 250 µL of ethyl acetate was added. The analysis was conducted using hydrogen as carrier gas, with a column flow rate of 4.5 mL/min and a total flow rate of 50 mL/min. The detector gases included air at 400 mL/min, hydrogen at 40 mL/min, and nitrogen at 45 mL/min. The AGILENT ChemStation software was used to automatically calculate the content in percentage, excluding the peaks of the silylation reagent. Any peaks not listed in the calibration table were disregarded, and the chromatogram was visually inspected for foreign components. Calibration and verification were performed according to the specified frequency, using reference samples.

### FT-IR spectroscopy:

The IR spectra were recorded with an FT-IR spectrometer Alpha II from Bruker at room temperature. The ATR (diamond) measurement principle was used. The spectrum was recorded from 400 cm⁻¹ to 4000 cm⁻¹. The analysis of the spectrum was enabled by the OPUS 7,5 and Origin software. The absorption was given in cm-1. The band at 1512 cm⁻¹ for BPA was considered, which represents the HCH bending vibration.

### Materials:

Sodium hydroxide (50 wt.% NaOH in H₂O) and methanol (98 %) were purchased from Fischer Scientifics. Dimethyl carbonate (99 %) and toluene (≥99.5) were purchased from Sigma Aldrich.

### PC composition 1:

A) 69.7 wt.% of polycarbonate based on bisphenol A
B) 29.9 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 0.4 wt.% of additives

### PC composition 2:

A) 49.05 wt.% of polycarbonate based on bisphenol A
B) 49.05 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 1.9 wt.% of additives

### PC composition 3:

A) 42.75 wt.% of polycarbonate based on bisphenol A
B) 42.75 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 14.5 wt.% of additives

### PC composition 4:

A) 44.3 wt.% of polycarbonate based on bisphenol A
B) 44.3 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard
C) 11.4 wt.% of additives

### PC composition 5:

A) 36.8 wt.% of polycarbonate based on bisphenol A
B) 36.8 wt.% of ABS made by bulk polymerization having an acrylonitrile:butadiene:styrene ratio of 23:10:67 and 24.5 wt.% of poly(methyl methacrylate) with 1 wt.% methyl acrylate content (Plexiglas^{®} 8H, Röhm). The weight average molecular weight of the free styrene acrylonitrile copolymer is 160,000 g/mol (measured by gel permeation chromatography (GPC) in tetrahydrofuran against a polystyrene standard. The weight average molecular weight of the poly(methyl methacrylate) is 147,000 g/mol (measured by gel permeation chromatography (GPC) in tetrahydrofuran under PMMA calibration).
C) 1.9 wt.% of additives

### PC composition 6:

This composition consists of recovered end-of-life material. It was analyzed and its composition was determined as follows: potassium bromide pellets were prepared from the ground original, and infrared spectra was recorded in transmitted light. Using a calibration of known polycarbonate contents, the PC content could be determined. Furthermore, a defined amount of the composition was depolymerized by means of sodium methoxide under reflux. The residue was separated from the solution, washed and dried. Using IR, the residue was found to be essentially PMMA due to the presence of the ester stretching frequency. The amount of PMMA was quantified by gravimetry and set as component B). The resulting content of the single components was found to be approximately as follows:
A) 36 wt.% of polycarbonate based on bisphenol A
B) max. 64 wt.% of poly(methyl acrylate)/poly(methyl methacrylate)
For the depolymerization of this material, the maximum possible amount of component B) was considered. This means that everything that depolymerized was assumed to be component A) and the whole rest of the sample which was not component A) was assumed to be component B).

### PC composition 7:

This composition consists of recovered end-of-life material. It was analyzed and its composition was determined as follows: potassium bromide pellets were prepared from the ground original, and infrared spectra were recorded in transmitted light. Using a calibration of known polycarbonate contents, the PC content could be determined. The rest of the IR spectrum gave indication to the presence of ABS as confirmed by the presence of the C-N stretching vibration at v = 2240 cm⁻¹. The content of the single components was found to be approximately as follows:
A) 75 wt.% of polycarbonate based on bisphenol A
B) max. 25 wt.% of ABS
For the depolymerization of this material, the maximum possible amount of component B) was considered. This means that the whole rest of the sample which was not component A) was assumed to be component B).

### PC composition 8:

A) 81.95 wt.% of polycarbonate based on bisphenol A
B) 8.65 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 9.4 wt.% of additives

### PC composition 9:

A) 60.19 wt.% of polycarbonate based on bisphenol A
B) 38.51 wt% of ABS comprising ABS made by bulk polymerization having an acrylonitrile: butadiene: styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 1.3 wt.% of additives

### PC composition 10:

A) 81.95 wt.% of polycarbonate based on bisphenol A
B) 8.65 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 9.4 wt.% of additives

### Depolymerization:

Inventive Example 1: In a 1 L reactor, 280.80 g of ground PC composition 1, 259.20 g of methanol (MeOH) and 1.20 g sodium hydroxide (NaOH 50 %) in water as a catalyst were added. The reaction mixture was stirred at 400 rpm and heated to 110 °C for 240 minutes or until a constant bisphenol A (BPA) concentration was reached. The BPA concentration was monitored by online-IR. The reaction solution was then cooled to ≤ 25 °C.

Table 1 shows the different polycarbonate compositions which were used according to the above given depolymerization scheme. Comparable reaction setups as given for inventive example 1 above were used. If dimethyl carbonate was actively added it was added together with PC, MeOH and the catalyst to the reactor. For all examples complete depolymerization of PC to BPA was ensured via online IR.

### Precipitation & Filtration:

The solution obtained from the decomposition of PC composition 1 of inventive example 1 was added to a stirred solution of methanol to give a fine non-sticky suspension. The resulting suspension was stirred for 3 minutes and then filtered through a glass frit. The precipitate was washed two times with methanol (V (filter cake)/V(MeOH) = 1/1. The filtrate was used in the following step of methanol/DMC evaporation.

### Methanol/DMC evaporation:

The solvent (methanol/DMC) was removed from the filtrate (containing BPA) using a rotary evaporator.

### Liquid-liquid Extraction & Crystallization:

The BPA-rich residue remaining after the methanol/DMC evaporation was combined with toluene and with water. The mixture was neutralized by addition of an acid. The water phase and toluene phase were separated at 85 °C through a separation funnel. The toluene fraction was cooled down to a temperature of about 5 °C; under these conditions the bisphenol A crystallizes from the solvent making it possible to selectively separate the bisphenol A crystals through filtration. For the filtration a paper prepleated filter (type Whatman 595 ½) with pore size 4 - 7 µm was used. The resulting residue was combined once again with toluene at 85 °C, cooled to 5 °C and the crystals were filtered once again. The crystals were dried in vacuum oven at 100 °C for 2 hours. B
Yield isolated BPA: 147.3 g (84%)
Purity of BPA (measured by GC): 99.87%

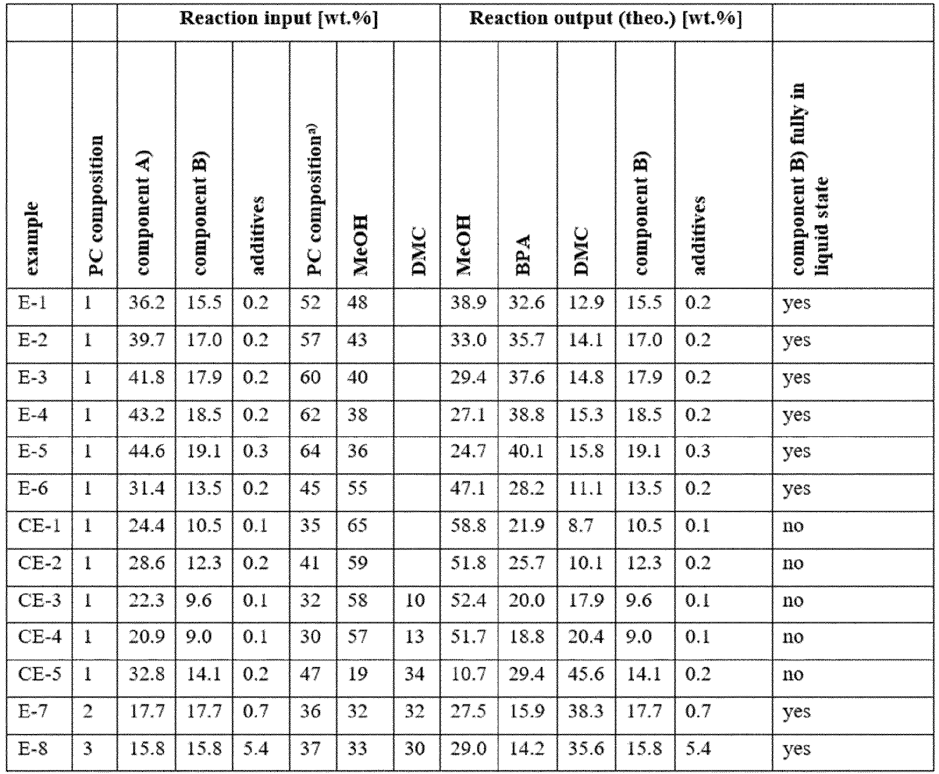

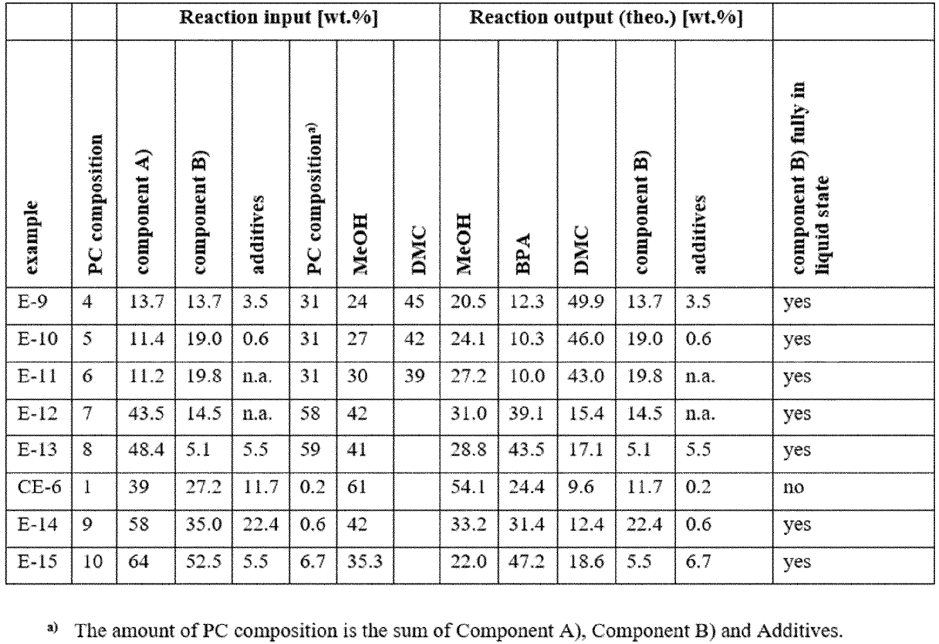

As can be seen from table 1, only when using the inventively defined conditions (a) to (c) component B) is in the liquid state after performing step (ii).

### Washing experiments:

### Inventive experiment E-1:

The reaction product of inventive example E-1 after depolymerization (inventive step (ii)) was added to a stirred solution of MeOH resulting in the formation of a suspension. The suspension was filtered and the BPA content of the solid was analyzed by HPLC to quantify the theoretical loss of BPA yield without washing (cf. Table 2, indicated there as "without washing"). The solid ABS residue was washed with MeOH (v/v of MeOH/wet ABS residue = 1/1) dried at 100°C in the vacuum oven for 2 hours and the BPA content in the ABS residue was analyzed by HPLC to quantify the theoretical loss of BPA yield after 1^{st} washing step (cf. Table 2 1^{st} washing step). The resulting ABS residue was mixed with MeOH (v/v of MeOH/wet ABS residue = 5/1) and was stirred for 10 minutes and filtered. The BPA content of the ABS residue (dried) was analyzed by HPLC to quantify the theoretical loss of BPA yield after 2^{nd} washing (cf. Table 2, 2^{nd} washing step).

For this example loss of BPA yield was calculated as follows: Loss of BPA yield = m(BPA in ABS)/ m(BPA total)
$Loss of BPA yield \left[wt%\right] = \frac{m \left(BPA in ABS\right)}{m \left(BPA total\right)}$ Where m(BPA in ABS) is the mass of BPA in the ABS residue after filtration which was determined by HPLC-MS and m(BPA total) is the theoretical mass of BPA obtained after complete conversion of component A to BPA.

### Comparative experiment CE-6:

After the depolymerization reaction (inventive step (ii)) as described above, the resulting reaction solution at 25 °C remained biphasic containing a clear and low viscous upper layer (MeOH phase) and a white, high viscous and non-flowable bottom layer (ABS phase). The BPA content of the bottom layer was analyzed by ¹H NMR spectroscopy to quantify the theoretical loss of BPA yield without washing (cf. Table 2, without washing).

The upper layer (MeOH phase) of the biphasic reaction mixture was removed by decantation. MeOH (v/v of MeOH/bottom layer = 5/1) was added to the ABS-rich phase and the mixture was mixed using a orbital shaker for 10 minutes. The upper layer (MeOH phase) was separated by decantation and the BPA content of the bottom layer was analyzed by ¹H NMR spectroscopy to the quantify the theoretical loss of BPA yield after the 1^{st} washing step. (cf. Table 2, 1^{st} washing step). The washing step was repeated followed by analysis of the BPA content of the bottom layer by ¹H NMR spectroscopy to quantify the theoretical loss of BPA yield after the 2^{nd} washing step. (cf. Table 2, 2^{nd} washing step).

For this example loss of BPA yield was calculated as follows: $Loss of BPA yield \left[wt%\right] = \frac{m \left(BPA in ABS\right)}{m \left(BPA total\right)}$

Where m(BPA in ABS) is the mass of BPA in the ABS rich phase after phase separation which was determined by ¹H NMR spectroscopy and m(BPA total) is the theoretical mass of BPA obtained after complete conversion of component A to BPA.

**Table 2**

| | Loss of BPA yield [%] | | |
|---|---|---|---|
| | Without washing | 1^{st} washing step | 2^{nd} washing step |
| E-1 | 14.3 | 3.8 | 0.9 |
| CE-6 | 38 | 36 | 23 |

As can be seen from Table 2 the inventive process provides for a higher BPA yield or a lower loss in BPA yield when performing the same numbers of washing steps and compared to a process in which the ABS was not in the liquid form to result a homogeneous solution and/or stable emulsion. As can be seen already the initial loss of BPA yield is much lower for the inventive example when compared to the comparative example. Moreover, the BPA can be removed more easily from the ABS which can be especially seen after the first washing step. In the inventive example the loss of BPA yield is reduced about 10 %, whereas for the comparative example this reduction is only 2 %.

## Claims

1. A process for the recovery of
(1) a dihydroxy compound and
(2) optionally a vinyl(co)polymer and/or a rubber-modified graft polymer from an article comprising the steps of
(i) bringing into contact an article, methanol, at least one catalyst and optionally dimethyl carbonate to form a reaction mixture, wherein the article comprises
A) polycarbonate comprising a structural unit derived from a dihydroxy compound and
B) at least one vinyl(co)polymer and/or a rubber-modified graft polymer,
(ii) performing a methanolysis reaction of the reaction mixture of step (i) yielding at least the dihydroxy compound from component A),
**characterized in that** component B) is in the liquid phase at least at the end of process step (ii),
wherein the total amount of any further component present in the reaction mixture in step (i) beside the article, methanol, at least one catalyst, optionally dimethyl carbonate, optionally water and optionally any impurity is at most 1 molar equivalent compared to 1 molar equivalent of the structural unit derived from the dihydroxy compound in the polycarbonate of component A).

2. The process according to claim 1, wherein the dihydroxy compound comprises at least one compound of formula (1) 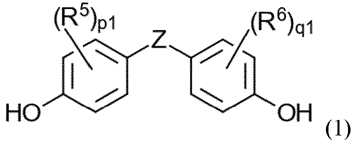
wherein each Z independently represents a single bond, -S(=O)₂-, -C(=O)-, -O-, -S-, - S(=O)-, -CH(CN)-, linear or branched C₁-C₆-alkylene which optionally comprises at least one carbonyl-group, optionally comprises at least one halogen atom and/or optionally is interrupted by at least one heteroatom, C₂-C₁₀-alkylidene which optionally comprises at least one carbon-carbon-double bond, optionally comprises at least one carbonyl-group and/or optionally comprises at least one halogen atom, C₅-C₁₂-cycloalkylene, wherein the cycloaliphatic group is fused to at least one further cycloaliphatic ring, C₅-C₁₅-cycloalkylidene wherein the cycloaliphatic group is optionally fused to at least one cycloaliphatic and /or at least one aromatic ring, C₇-C₂₀-aralkylidene, C₈-C₂₀-aralkylene, C₆-C₁₂-arylene optionally being fused to further aromatic rings which optionally may comprise at least one hetero atom, formula (B1-A), formula (B1-B), formula (B1-C) or formula (B1-D) 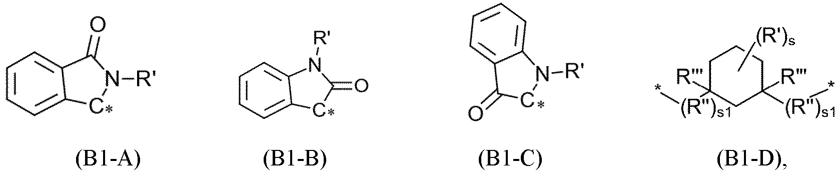
wherein in formulae (B1-A), (B1-B) and (B1-C) each R' independently represents a linear C₁-C₄-alkyl, branched C₃-C₄-alkyl, aralkyl or aryl and the "C*" indicates the quaternary carbon atom which is at the position indicated as "Z" in formula (3), and wherein in formula (B1-D) each R' independently represents a linear C₁-C₄-alkyl, branched C₃-C₄-alkyl, each R" independently represents a linear C₁-C₄-alkylene, branched C₃-C₄-alkylene, each R‴ independently represents H or linear C₁-C₃-alkyl, s is 0 to 2 and each s1 independently is 0 or 1, and the "*" indicate the bonds which link "Z" to the aromatic rings in formula (3),
each R⁵ and R⁶ independently represents H, C₁-C₁₈-alkyl, C₅-C₆-cycloalkyl, C₁-C₅-alkenyl-, C₁-C₁₈-alkoxy, phenoxy, halogen atom, C₆-C₁₈-aryl or C₇-C₁₈-aralkyl, and each of p1 and q1 is 1 to 4.

3. The process according to any one of claims 1 or 2, wherein the rubber-modified graft polymer is acrylonitrile-butadiene-styrene.

4. The process according to any one of claims 1 to 3, wherein the vinyl(co)polymer comprises structural units derived from at least one vinyl monomer selected from the group consisting of vinylaromatics, ring-substituted vinylaromatics, (C1-C8)-alkyl (meth)acrylates, vinyl cyanides and unsaturated carboxylic acids.

5. The process according to claim 4, wherein the vinyl(co)polymer is styrene-acrylonitrile copolymer and/or poly(methyl methacrylate).

6. The process according to any one of claim 1 to 5, wherein component B) is either a mixture of acrylonitrile-butadiene-styrene and styrene-acrylonitrile copolymer or it is poly(methyl methacrylate).

7. The process according to any one of claims 1 to 6, wherein the catalyst is any one selected from the group consisting of alkali metal or earth alkali metal hydroxides, alkali metal or earth alkali metal carbonates, alkali metal or earth alkali metal alkoxides, alkali metal oor earth alkali metal oxides, alkylamines, and pyridine.

8. The process according to any one of claims 1 to 7, **characterized in that** the impurity being optionally present in step (i) is selected from
(a) any impurity being introduced into step (i) by the article, methanol, the at least one catalyst, optionally the dimethyl carbonate and / or optionally the water and
(b) any impurity being introduced into step (i) by methanol, dimethyl carbonate and / or water, wherein the methanol, dimethyl carbonate and / or water is recovered from process step (ii), optionally purified and reused in process step (i).

9. The process of claim 8, **characterized in that** the impurity being introduced into step (i) by option (b) originates from any impurity and / or side product of the methanolysis reaction of step (ii) of the article.

10. The process according to any one of claim 1 to 9, **characterized in that** the impurity which is optionally present in step (i) is at most 15 wt.-%.

11. The process according to any one of claims 1 to 10 further comprising the steps of
(iii) precipitating component B) from the product as obtained after performing step (ii) to obtain a solid fraction comprising the vinyl(co)polymer and/or the rubber-modified graft polymer and a liquid fraction comprising the dihydroxy compound and
(iv) separating the solid fraction and the liquid fraction.

12. The process according to any one of claims 1 to 11, wherein the process is for the recovery of (2) the vinyl(co)polymer and/or the rubber-modified graft polymer.

13. The process according to claim any one of claims **11** or 12, further comprising the step of (v) isolating the dihydroxy compound.

14. A process for the production of a polymer comprising the step of polymerizing the dihydroxy compound recovered in the process according to any of the claims 1 to 13.

15. A process for the production of a polymer blend comprising the step of blending the vinyl(co)polymer and/or a rubber-modified graft polymer recovered in the process according to any of the claims 12 to 13 with a further polymer.
